(19) **Europäisches Patentamt**
**European Patent Office**
**Office européen des brevets**

(11) **EP 1 632 175 B1**

(12) **EUROPÄISCHE PATENTSCHRIFT**

(45) Veröffentlichungstag und Bekanntmachung des Hinweises auf die Patenterteilung:
**09.03.2016 Patentblatt 2016/10**

(51) Int Cl.:
*A61B 5/0452* *(2006.01)*      *A61B 5/00* *(2006.01)*
*A61N 1/362* *(2006.01)*      *A61N 1/37* *(2006.01)*

(21) Anmeldenummer: **05018875.4**

(22) Anmeldetag: **31.08.2005**

(54) **Signalverarbeitungsvorrichtung für physiologische Signale**

Signal processing apparatus for physiological signals

Dispositif de traitement de signaux physiologique

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **02.09.2004 DE 102004043005**

(43) Veröffentlichungstag der Anmeldung:
**08.03.2006 Patentblatt 2006/10**

(73) Patentinhaber: **Biotronik CRM Patent AG**
**6340 Baar/Zug (CH)**

(72) Erfinder:
- **Dörr, Thomas**
  **12437 Berlin (DE)**
- **Schneider, Peter**
  **12527 Berlin (DE)**
- **Weiss, Ingo, Dr.**
  **12435 Berlin (DE)**

(74) Vertreter: **Galander, Marcus et al**
**Biotronik SE & Co. KG**
**Corporate Intellectual Properties**
**Woermannkehre 1**
**12359 Berlin (DE)**

(56) Entgegenhaltungen:
**EP-A- 0 601 775**      **EP-A- 1 118 307**
**DE-A1- 2 836 890**      **DE-A1- 19 609 411**
**US-A1- 2002 193 696**

- **NOWAK BERND: "Pacemaker stored electrograms: teaching us what is really going on in our patients." Mai 2002 (2002-05), PACING AND CLINICAL ELECTROPHYSIOLOGY : PACE. MAY 2002, VOL. 25, NR. 5, PAGE(S) 838 - 849 , XP002356612 ISSN: 0147-8389 * Seite 838 - Seite 842 ***

## Beschreibung

Die Erfindung betrifft eine Signalverarbeitungsvorrichtung zur Verarbeitung physiologischer Signale sowie ein implantierbares medizinisches Gerät, insbesondere einen Herzschrittmacher mit einer derartigen Signalverarbeitungsvorrichtung.

Die Erfindung betrifft ganz allgemein das Problem, physiologische Signale in einer Art und Weise zu verarbeiten, dass die physiologischen Signale mit möglichst geringem Aufwand, insbesondere geringem Energieaufwand übertragen werden können. Ein besonderer Anwendungsfall ist die Übertragung eines intrakardialen beispielsweise mittels eines Herzschrittmachers oder eines implantierbaren Kardioverter/Defibrillators (ICD) aufgenommenen Elektrokardiogramms, welches zur Begutachtung durch einen Arzt oder zur Verarbeitung durch ein externes Gerät aus dem Implantat nach außen übertragen werden soll. Die Aufbereitung des intrakardial gemessenen Elektrokardiogramms sowie deren drahtlose Übertragung an ein externes Gerät erfordern Energie, die in dem Implantat nur begrenzt zur Verfügung steht. Ein hoher Energieverbrauch würde eine kurze Lebensdauer des Implantates mit sich bringen und dem Träger eines solchen Implantates unnötig viele Operationen zum Austausch des Implantates aufzwingen.

Vor diesem Hintergrund hat die Erfindung zum Ziel, eine Signalaufbereitung eines physiologischen Signals anzubieten, die selbst ressourcenschonend ist und es erlaubt, ein solches Signal mit wenigen Daten zu übertragen.

Erfindungsgemäß wird dieses Ziel durch eine Signalverarbeitungsvorrichtung der eingangs genannten Art erreicht, die eine Eingangsstufe zur Aufbereitung eines physiologischen Signals, insbesondere eines Elektrokardiogramms aufweist, die zur Bereitstellung eines aus dem physiologischen Signal gebildeten, auszuwertenden Signals mit einer alternierenden Polarität ausgebildet sowie zur Weitergabe des auszuwertenden Signals mit einer Auswertungsstufe verbunden ist. Die Auswertungsstufe ist ausgebildet, auf einen, dem physiologischen Signal zugeordneten Bezugszeitpunkt anzusprechen und für ein dem Bezugszeichen zeitlich zugeordnetes Signalsegment des auszuwertenden Signals jeweils einen Amplitudenwert für wenigstens einige in dem Signalsegment enthaltende charakteristische Kurvenpunkte (z.B. Signalmaxima und -minima, Inflexionspunkte etc.) zu erfassen und aus den so gewonnenen Amplitudenwerten einen die Form, d.h. die visuelle Erscheinung des jeweiligen Signalsegmentes beschreibenden Morphologiecode zu generieren, so dass die visuelle Erscheinung des Signalsegmentes mit Hilfe des Morphologiecodes ausreichend genau rekonstruiert werden kann.

Die Erfindung beruht auf der Erkenntnis, dass eine beispielsweise für einen Arzt ausreichend aussagekräftige visuelle Wiedergabe eines physiologischen Signals bereits dann zu rekonstruieren ist, wenn für ein jeweiliges Signalsegment die Amplituden der Signalmaxima und -minima sowie deren zeitliche Lage in Bezug auf einen Bezugszeitpunkt erfasst werden. Dementsprechend besteht der Kern der Erfindung darin, eine Signalverarbeitungsvorrichtung mit einer Auswertungsstufe vorzusehen, die auf einen Bezugszeitpunkt anspricht und für ein den Bezugszeitpunkt zugeordnetes Signalsegment die charakteristischen Kurvenpunkte, d.h. wenigstens die Signalminima und -maxima sowie deren Amplitudenwerte erfasst und aus diesen Werten einen Morphologiecode generiert. Das Ermitteln der Signalminima und -maxima und zwar vorzugsweise der absoluten Signalminima und -maxima zwischen jeweils zwei Nulldurchgängen des Signals unterscheidet die erfindungsgemäße Signalverarbeitungsvorrichtung von anderen, bekannten Signalverarbeitungsvorrichtungen, bei denen beispielsweise ein Elektrokardiogramm in regelmäßigen, kurzen Zeitabschnitten abgetastet wird und das so gewonnene Signal mit herkömmlichen, datenreduzierenden Verfahren, wie beispielsweise der aus der Bürodatenverarbeitung bekannten ZIP-Komprimierung weiterbearbeitet wird, um die Menge der zu übertragenden Daten zu reduzieren. Auch der mit der erfindungsgemäßen Signalverarbeitungsvorrichtung zu erzeugende Morphologiecode dient dazu, die Menge der zu übertragenden Daten zu reduzieren. Dies wird erreicht, indem nur die für die visuelle Darstellung entscheidenden Daten eines physiologischen Signals den Morphologiecode bestimmen und durch diesen repräsentiert sind.

Der Bezugszeitpunkt, auf den die Auswertungsstufe anspricht, ist vorzugsweise ein Zeitpunkt, zu dem sich ein physiologisches Ereignis, beispielsweise eine ventrikuläre Kontraktion in dem physiologischen Signal widerspiegelt, was in dem Beispielsfall in Form einer R-Zacke geschieht. Dementsprechend ist die Signalverarbeitungsvorrichtung vorzugsweise dazu ausgebildet, Elektrokardiogramme als physiologische Signale zu verarbeiten und auf jeweils eine R-Zacke in dem Elektrokardiogramm als Bezugszeitpunkt anzusprechen. Im Bezug auf eine Signalsegmentierung bedeutet dies, dass ein jeweiliges von der Auswertungsstufe bearbeitetes Signalsegment genau einem Herzzyklus zugeordnet ist.

Gemäß der Erfindung weist die Ausgangsstufe wenigstens einen Nulldurchgangsdetektor auf, der ausgebildet ist, Nulldurchgänge des auszuwertenden Signals innerhalb eines Signalsegmentes zu detektieren. Der Nulldurchgangsdetektor kann ein Nulliniendurchgangsdetektor sein und ist in einer bevorzugten Ausführungsvariante ein Nullbanddurchgangsdetektor, beispielsweise ein Schmidt-Trigger. Während ein Nulliniendurchgangsdetektor für einen Nulldurchgang von positiv nach negativ und umgekehrt denselben Referenzwert (Nullwert) anwendet, ist dieser Referenzwert für den Übergang von positiv nach negativ kleiner als für den Übergang von negativ nach positiv, so dass sich ein Nullband ergibt, das durch die beiden unterschiedlichen Referenzwerte bestimmt ist und welches eine Hysterese bewirkt.

Außerdem weist die Signalverarbeitungsvorrichtung einen Minimum- Maximumdetektor auf, der ausgebildet ist, ein jeweiliges absolutes Signalminimum oder -maximum des auszuwertenden Signals zwischen jeweils zwei benachbarten Nulldurchgängen hinsichtlich dessen Amplitude und vorzugsweise auch der relativen Position auf einer Zeitachse bezüglich eines Referenzsignals zu erfassen. Das Referenzsignal ist beispielsweise eine R-Zacke, deren Zeitpunkt des Auftretens den Bezugszeitpunkt bildet und deren Amplitude einen Bezugswert für das Erfassen der Amplituden der Signalmaxima und -minima im selben Signalsegment bildet. Das von der Auswertungseinheit erzeugte Auswertungssignal soll außerdem einen Skalierungsfaktor widerspiegeln der beispielsweise die Referenzsignalamplitude wiedergibt und damit den Faktor, mit dem die relativen Amplituden der Signalmaxima und -minima zu skalieren sind. Das so gewonnene Auswertungssignal bestimmt den zu gewinnenden Morphologiecode wenigstens teilweise mit.

Bereits angedeutet wurde, dass die Auswertungseinheit vorzugsweise dazu ausgebildet ist, das auswertende Signal, welches über die Eingangsstufe aus dem physiologischen Signal gewonnen wurde, segmentweise auszuwerten. Dies geschieht vorzugsweise derart, dass ein jeweiliges Signalsegment einen vor dem Bezugszeitpunkt beginnenden und nach dem Bezugszeitpunkt endenden Zeitraum umfasst. Der Zeitraum ist vorzugsweise derart festgelegt, dass er kürzer ist als die Zeitdauer zwischen zwei aufeinander folgenden Bezugszeitpunkten, so dass sich benachbarte Signalsegmente nicht überlappen.

Unter dem zuletzt genannten Aspekt ist es vorteilhaft, wenn die Eingangsstufe oder die Auswertungsstufe so ausgebildet sind, das physiologische Signal derart zu segmentieren, dass ein jeweiliges Signalsegment ein einem wiederkehrenden physiologischen Ereignis zugeordnetes Signal, insbesondere das Referenzsignal im vorgenannten Sinne, umfasst. Die Auswertungsstufe ist dann vorzugsweise dazu ausgebildet, für jedes Signalsegment einen jeweiligen Morphologiecode in Abhängigkeit des Auswertungssignals für das jeweilige Signalsegment zu generieren. Dazu dient - wie bereits angedeutet - das dem wiederkehrenden physiologischen Ereignis zuzuordnende Signal vorzugsweise als Referenzsignal. Das Referenzsignal ist beispielsweise eine jeweilige R-Zacke, wenn das physiologische Signal und das Auswertungssignal ein EKG ist. Die Referenzsignalamplitude ist vorzugsweise das absolute Maximum oder das absolute Minimum des auszuwertenden Signals innerhalb des jeweiligen Signalsegmentes und damit gleichzeitig vorzugsweise der Amplitudenwert des Referenzsignals. Entsprechend ist der Referenzsignalzeitpunkt der dem Referenzsignal als wiederkehrenden physiologischen Signal zuzuordnende Zeitpunkt des Auftretens im jeweiligen Signalsegment. Bezugswert sowohl für eine Analyse der (relativen) Amplituden der Signalmaxima und -minima innerhalb eines Signalsegmentes sowie auch für den Zeitpunkt von deren Auftreten ist somit vorzugsweise ein Referenzsignal, welches gleichzeitig dem absoluten Maximum oder absoluten Minimum des auszuwertenden Signals innerhalb eines Signalsegmentes entspricht. Dies erlaubt es, als Skalierungsfaktor lediglich einen einzigen, die Referenzsignalamplitude wiederspiegelnden Wert zu übertragen und im Übrigen die Nebenmaxima und -minima des auszuwertenden Signals innerhalb des Signalsegmentes durch relative Amplitudenwerte zu beschreiben, die auf die Referenzsignalamplitude bezogen sind. Gleichzeitig ist der Referenzsignalzeitpunkt vorzugsweise der Bezugszeitpunkt im vorgenannten Sinne.

Um den Bezugszeitpunkt zu gewinnen, kann die Auswertungseinheit in einer bevorzugten Ausführungsvariante mit einem Markersignalgenerator verbunden sein, der ausgebildet ist, ein physiologisches Signal auszuwerten und ein Markersignal zu generieren, welches ein dem physiologischen Signal zuzuordnendes Ereignis, beispielsweise ein kardiales Ereignis wie eine R-Zacke oder eine ventrikuläre Kontraktion, kennzeichnet, wobei die Auswertungseinheit ausgebildet ist, auf den durch ein Markersignal gegebenen Zeitpunkt als jeweiligen Bezugszeitpunkt anzusprechen. Auf diese Weise würde der Bezugszeitpunkt nicht intrinsisch aus dem auszuwertenden Signal gebildet, sondern extrinsisch durch die Markersignaleinheit, die als Eingangssignal ebenso ein aus dem physiologischen Signal gewonnenes Signal verarbeitet.

In einer bevorzugten Ausführungsvariante der Signalverarbeitungsvorrichtung ist deren Auswertungseinheit dazu ausgebildet, zur Bildung des Morphologiecodes die relativen Amplituden der Signalmaxima und -minima in wenige Amplitudenklassen in Abhängigkeit davon zu klassifizieren, wie die relative Größe der Signalmaxima und -minima in bezug auf die Referenzsignalamplitude ist. Die Auswertungseinheit bildet den Morphologiecode dann derart, dass der Morphologiecode einen Mustersubcode enthält, der wenige Werte annehmen kann und die Zugehörigkeit der jeweiligen Amplitude eines Signalmaximums und -minimums zu einer der Amplitudenklassen widerspiegelt. Zur Zuordnung der Amplituden der Signalmaxima und -minima zu einer jeweiligen Amplitudenklasse ist die Auswertungsstufe dazu ausgebildet zu bestimmen, ob die jeweilige Amplitude eines Signalmaximums oder -minimums im Verhältnis zum Referenzsignal ähnlich groß - also genauso groß oder nur wenig kleiner -, mittelgroß oder klein ist. Diese Zuordnung ist insbesondere dann vorteilhaft, wenn die Referenzsignalamplitude die größte Signalamplitude des auszuwertenden Signals innerhalb eines Signalsegmentes ist. Eine Zuordnung zu einer von insgesamt vier Amplitudenklassen "0", "klein", "mittel" und "groß" kann beispielsweise derart erfolgen, dass jedes Signalminimum oder -maximum mit einem Amplitudenwert, dessen Größe 75 bis 100% des Referenzsignalamplitudenwertes trägt, der Amplitudenklasse "groß" zugeordnet wird. Entsprechend wird ein relativer Amplitudenwert eines Signalminimums oder -maximums der Amplitudenklasse "mittel" zugeordnet, wenn deren Amplitudenwert 37,5 bis 75% des Referenzsignalamplitudenwertes betrifft. Die Amplitudenklasse "klein" wird einem Signalmaximum oder -minimum zugeordnet, dessen relativer Amplitudenwert zwischen 12,5 und 37,5% des Wertes der Referenzsignalamplitude beträgt. Die Amplitudenklasse "0" wird einem Signalmaximum oder -minimum zugeordnet, dessen relativer Amplitudenwert 0 bis 12,5% des Referenzsignalamplitudenwertes beträgt.

Die auf diese Weise gewonnene Information über die relativen Amplituden der Signalmaxima beziehungsweise -minima wird durch die Auswertungsstufe vorzugsweise derart verarbeitet, dass die Auswertungsstufe diese relativen Amplitudenwerte durch einen Ereigniscode wiedergibt, der für jedes Signalmaximum und -minimum eine Amplituden-Ziffer ($\alpha$) enthält, die einen von wenigen, vorzugsweise von vier Werten annehmen kann (die vier Werte entsprechen der Anzahl der Amplitudenklasse, beispielsweise "0", "klein", "mittel" und "groß"), und welche die Zugehörigkeit des jeweiligen Signalmaximums oder -minimums zu einer der Amplitudenklassen eindeutig beschreibt. Im verallgemeinerten Fall eines Kurvenpunkt-Detektors ist die ermittelte Ziffer eine Kennwert-Ziffer ($\alpha$).

Dazu ist die Auswertungsstufe vorzugsweise ausgebildet, den Ereigniscode so zu bilden, dass dieser wenige, vorzugsweise vier Amplituden-Ziffern enthält, die jeweils die Amplitude von insgesamt vier dem Referenzsignal benachbarten Signalmaxima oder -minima innerhalb des jeweiligen Signalsegmentes wiederspiegeln. Die vier Signalmaxima oder -minima können dabei zu beiden Seiten des Referenzsignals (links und/oder rechts) auftreten.

Allein der Ereigniscode bildet auf diese Weise bereits eine datenreduzierte Repräsentation eines jeweiligen Signalsegmentes. Eine weitere Datenreduktion lässt sich dadurch gewinnen, dass die Auswertungsstufe ausgebildet ist, aus dem Ereigniscode einen Mustersubcode (PPPPP) zu bilden, indem einer oder mehrere Ereigniscodes über eine Look-up-Tabelle einem Mustersubcodewert zugeordnet werden. Die Datenreduktion ergibt sich dann, wenn mehrere Ereigniscodes jeweils einem Mustersubcode zugeordnet werden. Es hat sich herausgestellt, dass 32 Mustersubcodes ausreichen, um alle in der Praxis auftretenden Signalsegmente eines EKG's als auszuwertendem Signal ausreichend genau beschreiben zu können.

Neben einer aus dem Ereigniscode gewonnenen Beschreibung eines jeweiligen Signalsegmentes in Bezug auf die relativen Amplituden der Signalmaxima und -minima umfasst der Mustersubcode vorzugsweise eine Vorzeichenziffer (S, im Folgenden auch Signum genannt), die das Vorzeichen der Referenzsignalamplitude widerspiegelt, also ob die Referenzsignalamplitude positiv oder negativ ist.

Außerdem umfasst der Morphologiecode vorzugsweise einen Signalbreiten-Subcode (WW), der vorzugsweise den zeitlichen Abstand zwischen einem ersten Signalmaximum oder -minimum und dem letzten Signalmaximum oder - minimum innerhalb eines Signalsegmentes oder alternativ auch die Dauer eines Signalsegmentes repräsentiert.

Um den Skalierungsfaktor zu repräsentieren, umfasst der Morphologiecode vorzugsweise einen Skalierungs-Subcode (AAAA), der den Skalierungsfaktor bildet und der die Referenzsignalamplitude als Absolutwert widerspiegelt.

Der Morphologiecode ist vorzugsweise ein Binärcode, der einen oder mehrere der folgenden Bestandteile (Subcodes) aufweist:

einen Signalbreiten-Subcode (WW), der vorzugsweise wenigstens zwei Binärstellen umfasst,

einen Vorzeichen-Subcode (S), der vorzugsweise eine Binärstelle umfasst,

einen Muster-Subcode (PPPPP), der vorzugsweise wenigstens fünf Binärstellen umfasst, und

einen Skalierungs-Subcode (AAAA), der vorzugsweise wenigstens vier Binärstellen umfasst.

Außerdem ist die Auswertungseinheit vorzugsweise dazu ausgebildet, dem Morphologiecode einen Zeitcode zuzuordnen und entsprechend hinzuzufügen.

Zur Signalaufbereitung umfasst die Eingangsstufe vorzugsweise einen Bandpassfilter, der ausgebildet ist, aus dem physiologischen Signal ein auszuwertendes Signal zu generieren, welches differenziert ist und entsprechend Nulldurchgänge aufweist.

Die Signalverarbeitungsvorrichtung ist vorzugsweise Bestandteil eines implantierbaren elektromedizinischen Gerätes, welches außerdem eine Telemetrieeinheit aufweist, die eingangsseitig mit der Signalverarbeitungsvorrichtung verbunden ist und dazu dient, den Morphologiecode drahtlos an ein externes Gerät zu übertragen.

In dem externen Gerät kann dann das physiologische Signal anhand des Morphologiecodes der Form nach rekonstruiert werden.

Für die vorstehende Beschreibung wurde eine Begrifflichkeit verwendet, der gemäß das physiologische Signal das Eingangssignal der Eingangsstufe ist, beispielsweise ein intrakardiales Elektrokardiogramm (iEGM). Das Ausgangssignal der Eingangsstufe und damit das Eingangssignal der Auswertungseinheit ist das auszuwertende Signal, welches durch die Eingangsstufe aus dem physiologischen Signal gewonnen wurde. Die Auswertungsstufe umfasst einen Maximum-/Minimumdetektor, dessen Ausgangssignal das Auswertungssignal ist (nicht zu verwechseln mit dem auszuwertenden Signal) welches die Signalmaxima oder -minima des auszuwertenden Signals innerhalb des Signalsegmentes widerspiegelt.

Als Bezugszeitpunkt für die Auswertung der Signalsegmente kann ein extrinsisch gewonnener Zeitpunkt gewählt werden, beispielsweise der durch ein Markersignal vorgegebene Zeitpunkt, oder ein intrinsisch gewonnener, aus dem auszuwertenden Signal abgeleiteter Zeitpunkt, beispielsweise der Zeitpunkt des Auftretens des größten Signalwertes innerhalb des auszuwertenden Signalsegmentes. Der größte Signalwert wird auch als Referenzsignal behandelt, dessen Amplitudenwert als Referenzsignalamplitude bezeichnet wird und dessen Zeitpunkt des Auftretens der Referenzsignalzeitpunkt ist und als intrinsisch gewonnener Bezugszeitpunkt dienen kann.

Ein Signalsegment ist ein Abschnitt des auszuwertenden Signals und jeweils einem Bezugszeitpunkt zugeordnet. Ein Signalsegment umfasst jeweils einen Zeitraum, der sich von Beginn bis Ende eines Signalsegmentes erstreckt.

Das Referenzsignal sowie weitere Signalmaxima oder -minima werden im Folgenden auch als Zacken bezeichnet. Eine zentrale Signalzacke bildet vorzugsweise das Referenzsignal. Die Nebenmaxima und -minima innerhalb eines Signalsegmentes bilden Satellitenzacken.

Die Erfindung soll nun im Folgenden anhand eines Ausführungsbeispiels näher erläutert werden. Dabei wird auf Figuren Bezug genommen, von denen

Figur 1: die Festlegung signifikanter Signalzacken illustriert;

Figur 2: eine Übersicht über eine typische Häufigkeitsverteilung von Morphologien intrakardialer Elektrokardiogramme gibt;

Figur 3: 32 typische Muster von Signalsegmenten eines iEGM's widerspiegelt, die ausreichen, ein iEGM hinreichend genau zu beschreiben;

Figur 4: ein Blockschaltbild eines beispielhaften Übertragungskanals als Eingangsstufe für die Signalverarbeitungsvorrichtung;

Figur 5: ein Blockschaltbild einer beispielhaften Auswertungsstufe für die Signalverarbeitungsvorrichtung;

Figur 6: ein Blockschaltbild eines typischen Markersignalgenerators;

Figur 7: ein Signalflussdiagramm, welches die Betriebsweise der beschriebenen Signalverarbeitungsvorrichtung und das Gewinnen des Morphologiecodes repräsentiert;

Figur 8: ein typisches Analysefenster für die Behandlung von Markersignalereignissen (ME) und Nicht-Markersignalereignissen (NME);

Figur 9: eine Darstellung der Rekonstruktion der Morphologie eines Elektrokardiogramms aus dem Morphologiecode;

Figur 10: ein Beispiel für ein Original eines Elektrokardiogramms sowie das entsprechende Rekonstrukt nach Auswertung des Morphologiecodes; und

Figur 11 a bis i: die Rekonstruktion des Verlaufs des codierten physiologischen Signals.

Die vorgestellte Signalverarbeitungsvorrichtung verwirklicht ein Verfahren, welches es erlaubt, aus einem auszuwertenden Signal gewonnene Parametersätze zu codieren und zu decodieren, die den Funktionszustand des Herzens in einem Zeitfenster (Signalsegment des auszuwertenden Signals) um einen bestimmten Bezugszeitpunkt (Ereignisgetriggert oder geplant) in sehr kompakter Form beschreiben und geht damit über bekannte Signalverarbeitungsvorrichtungen wie z.B. aus DE 2 836 890, EP 1 118 307 oder EP 0 601 775 U hinaus.

Die Arbeitsweise der Signalverarbeitungsvorrichtung beruht auf der Extraktion charakteristischer Merkmale aus Ereignis-diskreten kardialen Messsignalen, die für eine diagnostische Analyse oder/und Therapiesteuerung genutzt werden sollen. Die Definition der morphologischen Merkmale sowie deren Codierung orientieren sich dabei an den Prinzipien visueller Wahrnehmung. Ein Grundgedanke des Verfahrens ist es, die vorkommenden Signalmorphologien durch einen endlichen (ggf. sehr reduzierten) Satz repräsentativer Signalmuster bei der Rekonstruktion approximieren zu können. Das Verfahren kann auf zeitbegrenzte Signalausschnitte (Ereignissequenz) angewendet werden, es ist aber auch online-fähig und zur kontinuierlichen Codierung von Einzelereignissen geeignet.

Typische physiologische Signale, insbesondere kardiale Messsignale, für die das Verfahren eingesetzt werden kann, sind:

Ableitungen intrakardialer Elektrogramme (z.B. Tip-Ring IEGM, MAP, VER, farfield-IEGM)

Intrakardiale/Intrathorakale Impedanzsignale

Blutdrucksignale etc.

Die Signalquellen können ein- oder mehrkanalig sein. Im erweiterten Sinne ist das Verfahren auf beliebige Ereignis-diskrete Signale übertragbar (z.B. Atmung (Minute-Ventilation), bestimmte EEG-Wellen sowie Nervensignale, rhythmische Drüsenfunktionen, Schluckreflex, Lidschlag, usw.)

Mögliche Anwendungen des Verfahrens für Ereignis-diskrete Signale:

Optimierte Speicherung (Datenkompression, mit einem Speicherbedarf von vorzugsweise weniger als 2.5 Byte/Ereignis bei einem max. Abstand von 64 Sample-Intervallen zwischen 2 Ereignissen)

Optimierte Übertragung (reduzierte Datenrate für schmalbandige Übertragungskanäle, z. B. Home Monitoring oder andere Applikationen der Telemedizin)

Semantische Datenstrukturierung (als Vorverarbeitung für Rechnergestützte Auswertung wie automatisierte Kontext-bezogene Diagnose in einer Home Monitoring Zentrale)

Teil des Rückkopplungszweigs eines therapeutischen Reglers (Closed-Loop-Therapie: z.B. hinsichtlich Frequenzanpassung; Automatic Capture Control, Automatic AA-, AV-, VV-delay, Mode-Switch Control).

Farfield-Unterdrückung und Rhythmusdiskriminierung (z.B. in ICDs SVT/VT) basierend auf morphologischen Informationen (Kontext-Analyse über Phrasen aus morphologischen Deskriptoren)

Randbedingungen/Anforderung für die Anwendbarkeit

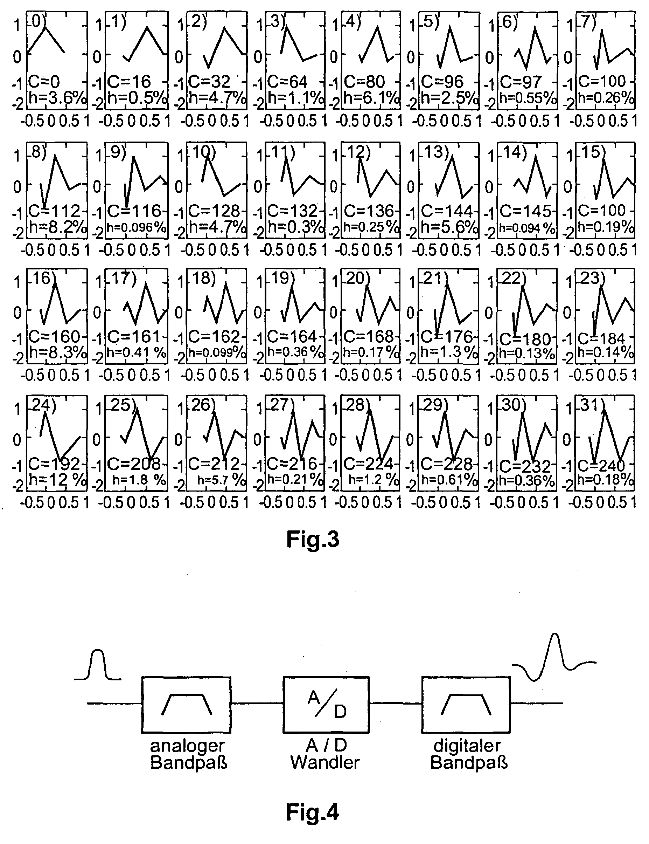

Die kompakte Signalbeschreibung wird durch Ausnutzung folgender Randbedingungen erzielt, die durch eine Eingangsstufe (siehe Figur 4) der Signalverarbeitungsvorrichtung sichergestellt werden:

Natur des auszuwertenden Signals:

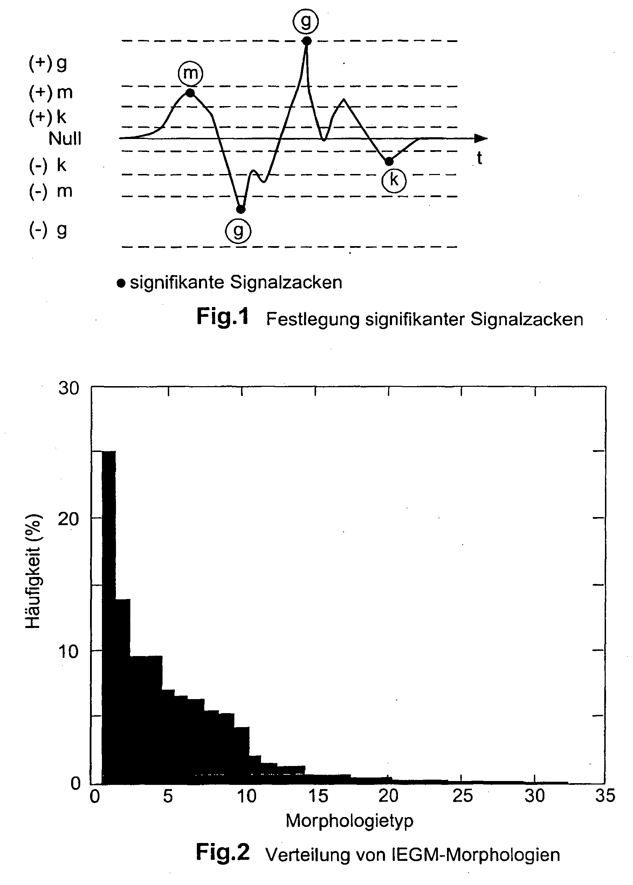

Das Verfahren setzt voraus, dass sich die signifikante morphologische Information aus den Extrema des Signalverlaufs herleitet, die jeweils zwischen 2 Nullbanddurchgängen bestimmt werden. Für ein jeweiliges zu analysierende Signalsegment wird dabei angenommen, dass es von einer Nulllinie startet und auch wieder auf der Nulllinie endet. Befinden sich zwischen den angrenzenden Nullbanddurchgängen mehrere lokale Extrema, wird nur das absolute Extremum im jeweiligen Bereich als relevant betrachtet; die anderen Spitzen werden vernachlässigt. Dies hat zur Folge, dass die Abfolge der somit bestimmten relevanten Extrema sich dadurch auszeichnet, dass deren Polarität stets alternierend ist. Diese Information (Alternanz der Polarität) ist daher redundant und muss nicht übertragen werden (Figur 1).

Ferner setzt das Verfahren voraus, dass ein jedes der betrachteten Signalsegmente durch einen entsprechenden Repräsentanten aus einer endlichen Anzahl verschiedener charakteristischer Morphologien (Ereignismuster) ausreichend gut approximiert werden kann. Eine typische Häufigkeitsverteilung für Morphologien rechtsventrikuläre Tip-Ring IEGMs und typische Muster ist in Figur 2 und 3 angegeben. Somit reichen diesem Verfahren zur Codierung von IEGM-Signalen 32 Mustern aus.

Signalkonditionierung in der Eingangsstufe

Die in Figur 4 beispielhaft abgebildete Eingangsstufe generiert aus dem jeweiligen physiologischen Signal, beispielsweise einem intrakardial abgeleitetem Signal, ein für die weitere Auswertung durch eine Auswertungsstufe geeignetes auszuwertendes Signal.

Intrakardial abgeleitete Signale, die durch ein Implantat aufgezeichnet werden, sind stets Bandpass-gefiltert. Der Hochpassanteil resultiert aus der kapazitiven Ankopplung des Geräts an das Gewebe um einen schädlichen DC-Stromfluss zu vermeiden. Der Tiefpass dient als Antialiasing-Filter für die anschließende Abtastung (siehe Figur 4, analoger Bandpass). Als Vorverarbeitung für die Sensingstufe eines kardiotherapeutischen Implantats wird das digitalisierte Signal einer weiteren Bandpassfilterung unterzogen (siehe Figur 4, digitaler Bandpass), dessen stark differenzierender Effekt auf das Signal typischerweise einen gewünschten Signalverlauf mit hinsichtlich der Polarität alternierenden Spitzen zur Folge hat. Für die in dieser Weise vorverarbeiteten Signale sind die Anforderungen an die Natur der Signalquelle in sehr guter Näherung erfüllt.

Ist dies nicht der Fall, kann diesem Verfahren jedoch eine entsprechende Signalkonditionierung künstlich vorgeschaltet werden, die nach der Rekonstruktion wieder zu kompensieren ist. Dies ist zwar nur näherungsweise möglich, jedoch für die meisten praktischen Anforderungen ausreichend. Eine Verbesserung der Ergebnisse kann durch Übertragung zusätzlicher Signal-Metriken (z.B. Signalfläche) erzielt werden.

Verwendung von Informationen, die im Implantat bereits verfügbar sind:

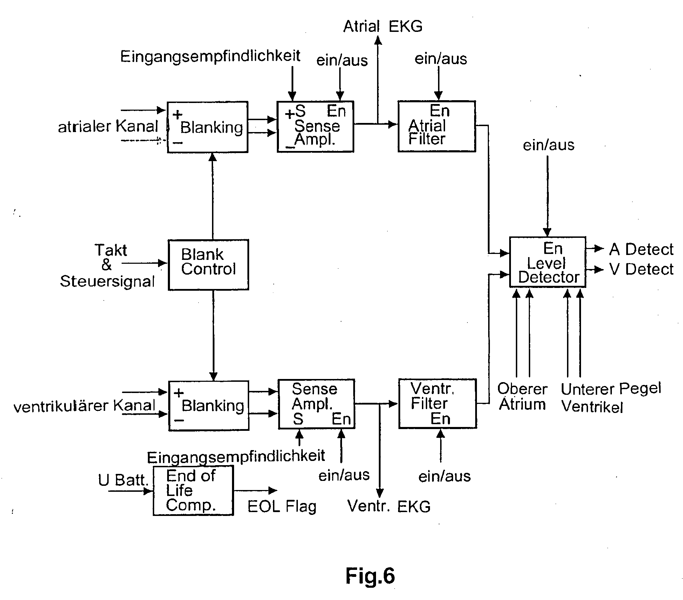

Seine Therapieentscheidung leitet ein kardiotherapeutisches Implantat aus Informationen über die momentan vorliegende Herzaktivität her, so dass damit bereits eine Segmentierung des Signals vorliegt. Die Segmente sind durch sog. "Marker" gekennzeichnet, die ein an sich bekannter Markersignalgenerator, wie er in Figur 6 dargestellt ist, generiert. Ein solches Markersignal liegt an den Ausgängen des Level-Detektors aus Figur 6, die mit "V-Sense" und "A-Sense" bezeichnet sind an, sobald ein ventrikuläres oder ein atriales Ereignis erfasst wurde.

Es folgt nun eine Beschreibung der Arbeitsweise der Auswertungsstufe der Signalverarbeitungsvorrichtung. Zunächst ein Überblick über die Signalverarbeitungsvorrichtung und das Verfahren:

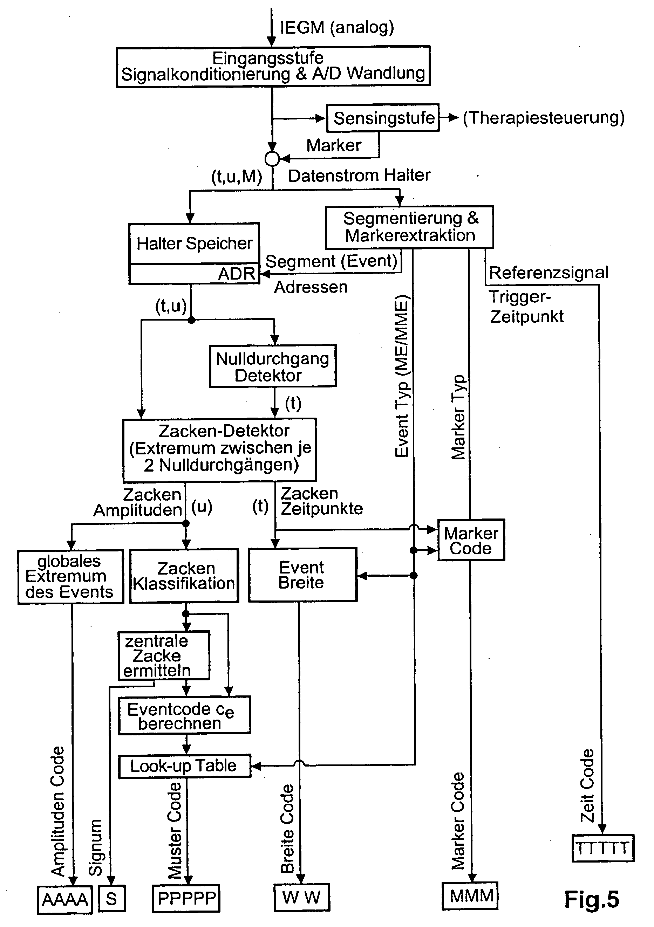

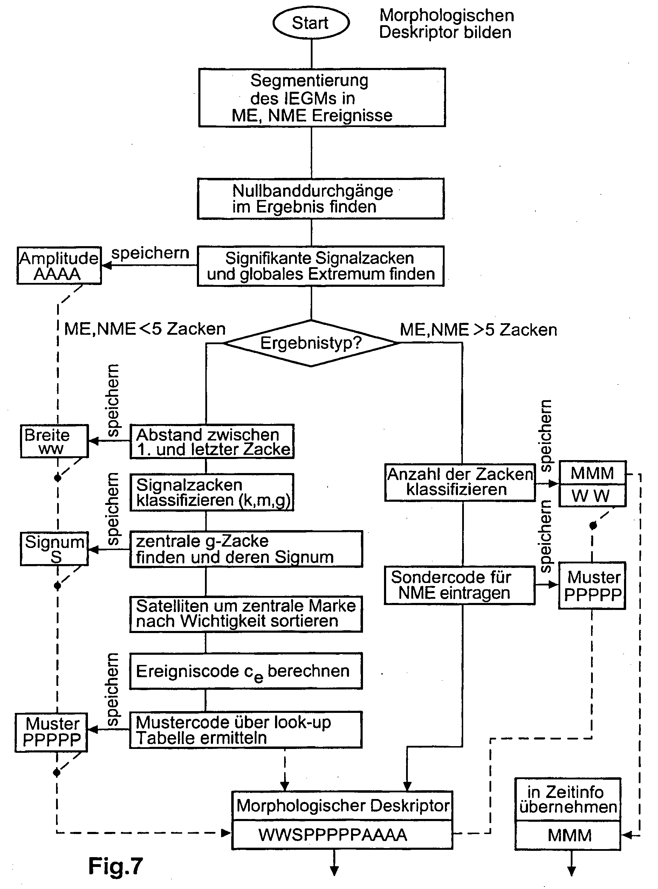

Der Eingangsstufe ist eine Auswertungsstufe nachgeschaltet, die in Figur 5 in einem schematischen Blockschaltbild dargestellt ist und die nach folgendem, in Figur 7 anhand eines Flussdiagramms dargestellten Verfahren arbeitet:

Das Verfahren codiert/decodiert Signale auf einer Ereignis-diskreten Basis. Die Codierungsvorschrift ist für geringe Rechenleistung ausgelegt und in einer bestimmten Implementierung besonders zur Realisierung als State-Machine oder als Assembler-Code optimiert. Es ist daher möglich, dieses Codierverfahren in einem medizinischen Implantat zu realisieren. Der Aufwand verlagert sich zum Decodierverfahren, das auch zur Einbeziehung von Expertenwissen (z.B. in Form eines Expertensystems) konzipiert ist.

Im Folgenden wird nun die Codierung beschrieben, zu deren Durchführung die Signalverarbeitungsvorrichtung ausgebildet ist.

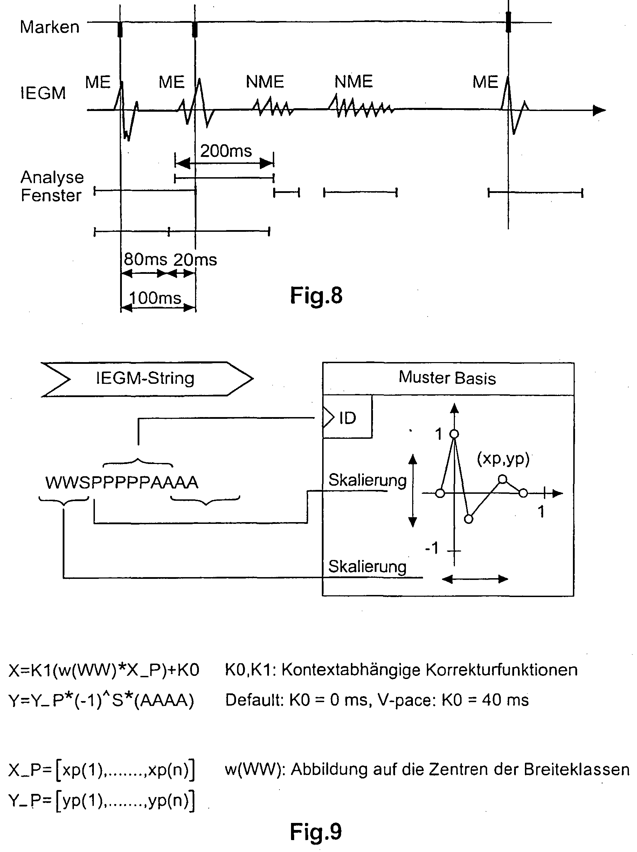

Ein Signalflussdiagramm der Codierung ist in Figur 7 skizziert. Vorbereitend für die Codierung wird das Signal zunächst segmentiert. Es wird dabei zwischen ME- (Marker Event) und NME-Segmenten (Non Marker Event) unterschieden. ME Segmente befinden sich innerhalb eines festgelegten Zeitfensters um einen durch einen Marker gegebenen Bezugszeitpunkt (konkrete Realisierung: 40 ms davor bis 160 ms danach), wobei die Fenstergrenzen ggf. so angepasst werden, dass sich 2 benachbarte ME-Segmente nicht überlappen (anteilige Verkürzung abhängig vom Abstand der benachbarten Marker, konkrete Realisierung: Aufteilung des Abstands zwischen den benachbarten Markern in Verhältnis 80% / 20%). NME-Segmente sind in den Bereichen außerhalb der ME-Fenster zu suchen, wobei zwischen 2 ME-Fenstern auch mehrere NME-Segmente gefunden werden können (Figur 8). Es handelt sich dabei z.B. um Herzaktivitäten, die im Hinblick auf die Therapiesteuerung durch die Sensingstufe als nicht relevant klassifiziert wurden, jedoch diagnostisch von Bedeutung sein können. Die Grenzen der NME-Segmente werden entweder durch ein ME Segment oder durch einen Signalabschnitt bestimmt, der innerhalb eines festgelegten Zeitfensters eine Signalenergie geringer als ein festgelegter Wert aufweist (konkrete Realisierung: Zeit 24 ms, Signalenergie=0).

Innerhalb der Signalsegmente (ME und NME) werden die Nullbanddurchgänge ermittelt und jeweils dazwischen das Extremum identifiziert (signifikante Signalzacke dieses Abschnitts siehe Figur 1). Für das zu analysierende Signalsegment wird dabei angenommen, dass es von der Nulllinie startet und auch wieder auf der Nulllinie endet. Durch dieses Verfahren erhält man eine Abfolge von Signalzacken, deren Polarität stets alterniert. Für die Rekonstruktion ist es daher ausreichend, nur die Absolutwerte der Amplituden und die Polarität einer ausgewählten Signalzacke zu codieren.

Für das weitere Vorgehen wird zwischen ME und NME unterschieden:

Zunächst soll die Verarbeitung von Markerevents (MEs) beschrieben werden.

Im Falle von ME werden eine max. Anzahl von N Signalzacken ab Segmentbeginn betrachtet (konkret N=5). Die Amplituden der so bestimmten Signalzacken werden über eine i.a. nichtlineare Abbildung klassifiziert (kA Klassen). In Anlehnung an die visuelle Wahrnehmung werden in der konkreten Realisierung die Amplitudenklassen "Null", "klein" (k), "mittel" (m) und "groß" (g) unterschieden (d.h. kA=4). Die Schwellwerte der Klassifizierung werden relativ zur größten Signalzacke (100 %) festgelegt (konkret: Klassengrenzen "Null"=0-12.5%, "klein"=12.5-37.5%, "mittel"= 37.5-75% und "groß"=75-100%). Diese Grenzen können fest oder programmierbar gestaltet werden. Die Amplitude (A) der größten Signalzacke (und damit des ME) wird in adäquater Auflösung gespeichert (konkret 4 bit).

Unter den Signalzacken höchster Klasse (i.a. können es mehrere sein) wird aus morphologischen Gesichtspunkten diejenige mit höchster Bedeutung ermittelt. Sie wird ferner "zentrale Signalzacke" genannt. In der konkreten Realisierung wird dabei unterschieden, ob die Anzahl (ng) der Signalzacken vom Typ g gerade (ng=2n) oder ungerade (ng=2n+1) ist. Im ungeradzahligen Fall erhält die mittlere in der Unterliste der g-Zacken den Rang der "zentrale Signalzacke". Im geradzahligen Fall, wird für die Signalzacken n und n+1 in der Unterliste der g-Zacken geprüft, welche davon in der Gesamtliste aller betrachteten N Zacken der Listenmitte am nächsten liegt und diese dann als zentral betrachtet. Besteht Gleichheit, wird für die linkere entschieden. Damit ist sichergestellt, dass stets möglichst viele der Zacken effektiv zur Berechnung des die Morphologie kennzeichnenden Ereigniscodes beitragen und folglich das Ereignis bestmöglich beschrieben wird. Für die zentrale Signalzacke wird das Signum (S) bestimmt und abgespeichert.

Der zeitliche Abstand zwischen der ersten und letzen der N Zacken wird ebenfalls über eine i. a. nichtlineare Abbildung klassifiziert (kB Klassen). Er verschlüsselt die Breite (W) des Ereignisses. Diese Grenzen können fest oder programmierbar gestaltet werden. In der konkreten Realisierung werden kB=4 Klassen definiert (Codierung mit 2 bit).

Zur Berechnung des Ereigniscodes ("Quantifizierung" der Morphologie) wird wie folgt vorgegangen:

Die Amplitudenklassen werden so bezeichnet, dass sie Ziffern ($\alpha$) einer Zahl in der Basis $\beta$ ($\beta$=kA) darstellen. D.h. der Wertebereich der Ziffern erstreckt sich von 0 bis kA-1. Damit wird eine Zahl in der Basis $\beta$ gebildet, wobei die Stellenordnung der Ziffern (d.h. der entsprechenden Zacken) gemäß ihrer Wichtigkeit festgelegt wird. Die zentrale Signalzacke hat stets die höchste Wichtigkeit. Diese Information ist redundant und muss demnach nicht übertragen werden. Die zentrale Signalzacke ist definitionsgemäß vom Typ g und ihr Vorzeichen ist bereits durch das Signum S beschrieben.

Die Unterschiede zwischen den Morphologien resultieren daher nur aus den Satelliten-Zacken, die das zentrale g umgeben. Die Wichtigkeit dieser Zacken sei über eine Abbildung a(i) beschrieben, die die Ziffern (stellvertretend für die Zacken) den entsprechenden Potenzen zuordnet.

Der Ereigniscode (Ce) ist demnach eine positive Zahl in der Basis $\beta$ mit bis zu N-1 signifikanten Stellen:

$$C_e = \sum_{i=0}^{N-2} \alpha_{a(i)} \cdot \beta^i \quad (1)$$

Durch geeignete Wahl der Abbildung a(i) kann erreicht werden, dass Ereignisse mit nahe beieinander liegenden Codes eine bestimmte morphologische Ähnlichkeit aufweisen. Damit wird auf den Raum der Morphologien eine Ordnungsrelation definiert. Dies ist eine ganz entscheidende Voraussetzung dafür, dass auch mit einem reduzierten Mustersatz der ganze Raum der Morphologien zumindest näherungsweise aber im Hinblick auf die Anzahl seiner Elemente vollständig beschrieben werden kann. Liegt in der reduzierten Musterbasis kein Muster vor, dessen Ereigniscode mit dem berechneten Code exakt übereinstimmt, wird zur Rekonstruktion das Muster hergenommen, dessen Ereigniscode sich hiervon am wenigsten unterscheidet.

In der konkreten Realisierung gestaltet sich die Abbildung a(i) wie folgt: Die Satteliten-Zacken werden alternierend von rechts und links der zentralen Signalzacke genommen und den Potenzen zugeordnet. Gestartet wird unmittelbar rechts der zentralen Signalzacke und die Wichtigkeit nimmt mit der Entfernung hiervon stets ab. Müssten dabei aufgrund der speziellen Lage der zentralen Signalzacke Positionen außerhalb der N betrachteten Zacken eines MEs angesprungen werden, sind die entsprechenden Koeffizienten in (1) gleich Null zu setzen.

Für die konkrete Realisierung ist $\beta$=4, wodurch eine vorteilhafte Implementierung in einem binären System möglich ist (in Hard- sowie Software). Bezeichnend für dieses Verfahren ist, dass die Ziffer 0 inmitten der Zahl nicht vorkommt. D.h. die Anzahl der möglichen unterschiedlichen Ereigniscodes ist wesentlich geringer als die Anzahl aller möglichen Zahlen, die in der Basis $\beta$ mit N-1 Stellen gebildet werden können. Für die konkrete Realisierung bedeutet das, dass für N=5 und kA=4 weniger als 256 unterschiedliche Ereigniscodes vorkommen (denn 3*4^3+3*4^2+3*4^1+3=255).

Im Falle der IEGM Signale kommen bestimmte Muster wesentlich häufiger vor als andere (siehe Bild 2), so dass auf seltene Muster in erster Näherung verzichtet werden kann, insbesondere, da die sichergestellte Ordnungsrelation eine gute Approximation durch ein anderes Muster ermöglicht. Im konkreten Fall ist es sinnvoll die Größe der Musterbasis auf 32 Repräsentanten zu reduzieren. Den Ereigniscodes die gemäß (1) für die Muster der Musterbasis zu errechnen sind, werden Mustersubcodes (P) zugeordnet, die aufgrund ihrer geringeren Anzahl auf kleineren Wortbreiten dargestellte werden können. Die Abbildung der Ereigniscodes auf die Menge dieser Mustersubcodes kann entweder durch eine auf der Ordnungsrelation basierenden Rechenvorschrift oder per Look-Up Tabelle erfolgen. Die Look-Up Tabelle ist dabei programmierbar (d.h. nicht "festverdrahtet"). Anstelle des für eine Signalzackensequenz eindeutigen Ereigniscodes ist der Code des Musters zu übertragen, das im Sinne der Ordnungsrelation dem Original am ähnlichsten ist (d.h. dessen Ereigniscode dem des Originals am nächsten liegt). Es handelt sich hierbei um eine verlustbehaftete Quellkodierung. Aufgrund der sehr ungleichen Häufigkeiten ist ferner zur optimierten Codierung (Abbildung von Ereigniscodes auf Mustercodes) die Anwendung eines Entropiecodierverfahrens (z.B. Hufmann) vorteilhaft.

Prinzipiell kann dieses Verfahren zur Codierung der Amplitudenmuster auch für die Muster adaptiert werden, die sich aus den Breiten der Einzelzacken ergeben. Dann sind die Zackenbreiten zu klassifizieren, Ziffern zuzuordnen und in ähnlicher Weise wie in (1) zu einem Morphologiesubcode für die Breiteninformation zu verrechnen. Ähnlich den zur Klassifikation der Amplituden vorgesehenen Amplitudenklassen können zur Klassifikation der Zackenbreiten Breitenklassen (kB) vorgesehen sein, die die relative Breite einer Einzelzacke innerhalb eines Signalsegmentes beschreiben.

Die Breite einer Einzelzacke lässt sich dann rekonstruieren, weil die Anzahl der Einzelzacken und ihre durch die Zuordnung zu einer der Breitenklassen gegebene relative Einzelzackenbreite bekannt sind und mit der durch den Signalbreiten-Subcode gegebenen Gesamtbreite eines Signalsegmentes oder eines Ereignisses verrechnet werden können. Der Morphologiecode ist dann um diese Breiteninformation zu ergänzen. Für die konkrete Realisierung der IEGM-Codierung stellte sich die Codierung der Zackenbreiten als nicht erforderlich heraus.

Der Morphologiecode fasst in geeigneter Aneinanderreihung die oben beschriebenen morphologischen Metriken eines jeden zu übertragenden Ereignisses zusammen. Im Falle der konkreten Realisierung wird der Morphologiecode wie folgt aufgebaut:

WWSPPPPPAAAA

Jedes der Symbole steht für 1 Bit und hat die im Text erläuterte Bedeutung. Diesem Morphologiecode kann formal noch eine weitere Information zugeordnet werden, die über die rhythmische Klassifikation des Ereignisses Auskunft gibt. Diese Information (wie z.B. VT Klasse) ist in kardiotherapeutischen Implantaten in der Regel an den Marker (Markertyp M) geknüpft und wird daher in der konkreten Realisierung bei der Zeitkodierung verschlüsselt.

Das Verfahren zur Ermittlung des Morphologiecodes ist dem der Vektorquantisierung zwar ähnlich, unterscheidet sich jedoch dadurch, dass es für Signal-blöcke unterschiedlicher Länge anwendbar ist, was für die konkrete Anwendung unverzichtbar ist.

Es folgt nun eine Beschreibung der Verarbeitung von Non-Marker-Events.

Non Marker Events mit bis zu N=4 Zacken werden in der konkreten Realisierung nach der gleichen Vorschrift wie Marker Events analysiert, die Codierung ordnet jedoch den Codefeldern des Deskriptors teilweise eine andere Bedeutung zu. Statt der für NMEs nicht verfügbaren Klassifikation hinsichtlich des Markertyps codiert M die Anzahl der Zacken und für N>4 gibt W in gröberen Klassen unterteilt die Anzahl der Signalzacken eines NMEs an. Für NMEs, die häufig höherfrequent schwingende Signalanteile darstellen, ist ebenso ein Maß für die Frequenz zu codieren; in der einfachsten Realisierung die binäre Information "schneller" bzw. "nicht schneller" als eine Bezugsfrequenz.

Schließlich ist vorzugsweise eine Zeitcodierung vorgesehen, die im Folgenden beschrieben wird.

Die Codierung der Zeitinformation ist für den allgemeinen Fall einer mehrkanaligen Speicherung/Übertragung konzipiert. Es wird dabei stets die Zeit zu dem im Codierverfahren zuletzt verarbeiteten Ereignis gemessen und codiert, unabhängig davon in welchem Kanal dieses auftrat. Die spätere Zuordnung ist aus dem Markertyp (M) eindeutig zu entschlüsseln. Dies hat den Vorteil, dass die Zahlenwerte für die zu codierenden Zeiten kleiner sind und folglich geringere Wortbreiten erforderlich sind. Dieser Vorteil ergibt sich insbesondere bei IEGM-Signalen aus unterschiedlichen Herzkammern, deren Ereignisse überwiegend zeitversetzt sind. Um im Falle langer Zeitintervalle zwischen Ereignissen einen Überlauf zu vermeiden, sieht das Verfahren einen Überlaufcode vor, der einen weiteren ergänzenden Zeitwert ankündigt. Durch mehrfach hintereinandergeschaltete Überlaufcodes können beliebig lange Zeitintervalle kodiert werden.

Der Zeitcode ist in der konkreten Realisierung wie folgt aufgebaut:

[Überlaufcode, ..., Überlaufcode],MMMTTTTT

wobei jedes Symbol für je ein Bit des Markertyps (M) bzw. des Zeitintervalls (T) steht.

Das Verfahren kann sowohl in kausaler als auch akausaler Richtung arbeiten. Der letztere Fall setzt voraus, dass die Rohdaten bereits zwischengespeichert sind und ist dann von Interesse, wenn die Vorgeschichte, also der Signalverlauf vor dem Eintreten eines definierten Triggers, zu codieren ist.

Die so gewonnenen Daten zur Codierung eines physiologischen Signals können über einen Datenstrom an ein externes Gerät übertragen werden. Im Folgenden wird ein beispielhafter Aufbau eines solchen Datenstromes beschrieben.

In einer i. a. mehrkanalig gemischten Darstellung stellt der Datenstrom eine Aufeinanderfolge von Zeit- und Morphologiedaten dar, wobei die morphologischen Deskriptoren optional sind. Im Falle der konkreten Realisierung werden nur für die ventrikulären IEGM-Signale morphologische Deskriptoren codiert, während sich die atrialen Daten auf rhythmologische Informationen beschränken. Ob einer (obligatorischen) Zeitinformation ein morphologischer Deskriptor folgt, ist am Markertyp zu erkennen der stets Bestandteil der Zeitinformation ist. Zur Gewährleistung der Eindeutigkeit wird in der konkreten Realisierung der Datenstrom grundsätzlich mit einer Zeitinformation begonnen.

Ein derartiger Datenstrom kann von einem externen Gerät empfangen und dort wieder in eine Darstellung eines physiologischen Signals gewandelt werden. Dazu ist das externe Gerät ausgebildet, den Datenstrom wie folgt zu rekonstruieren.

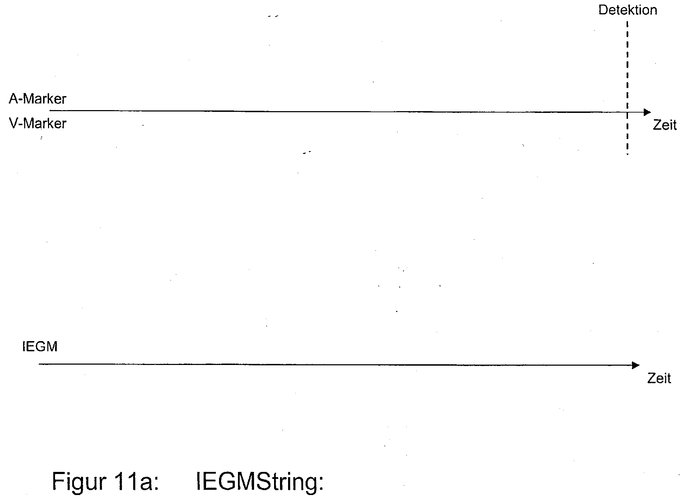

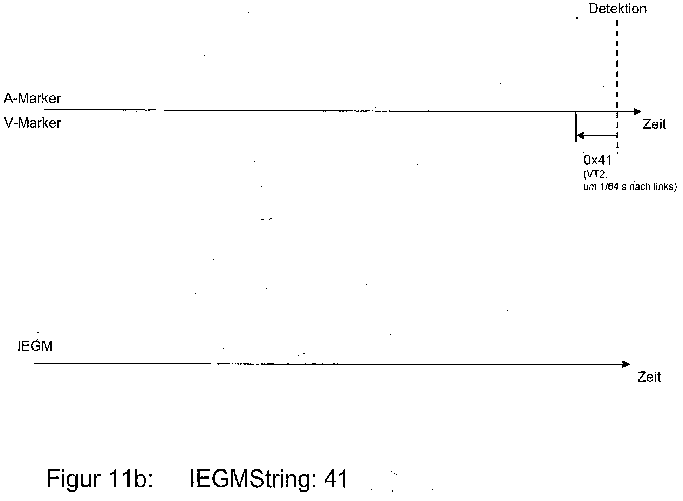

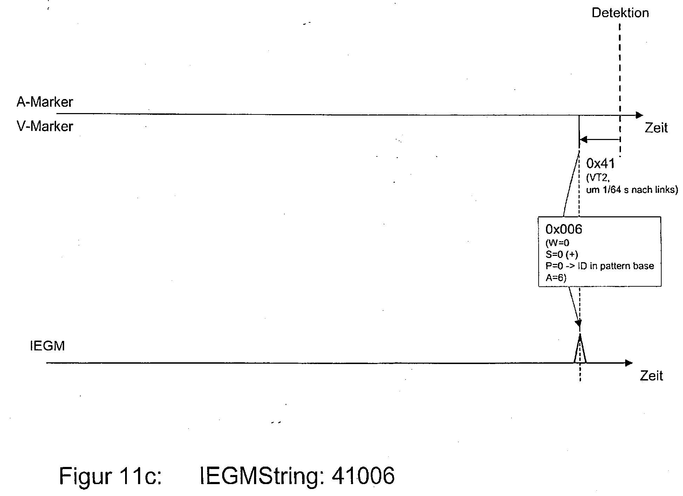

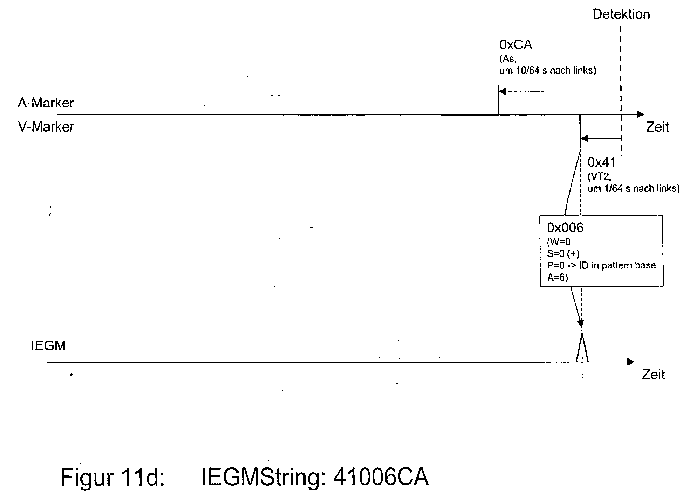

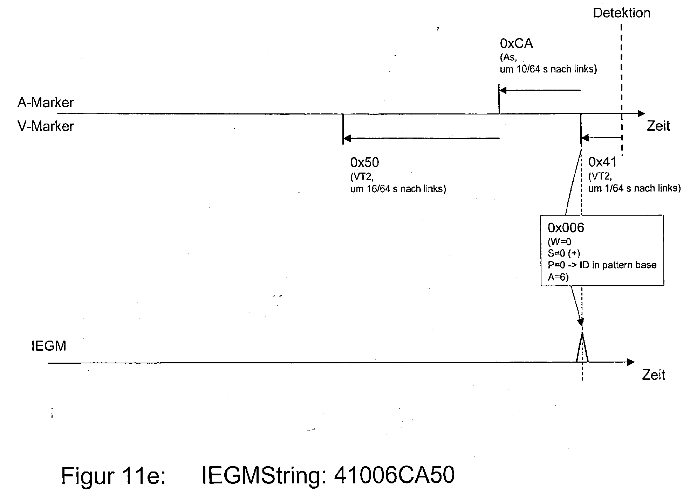

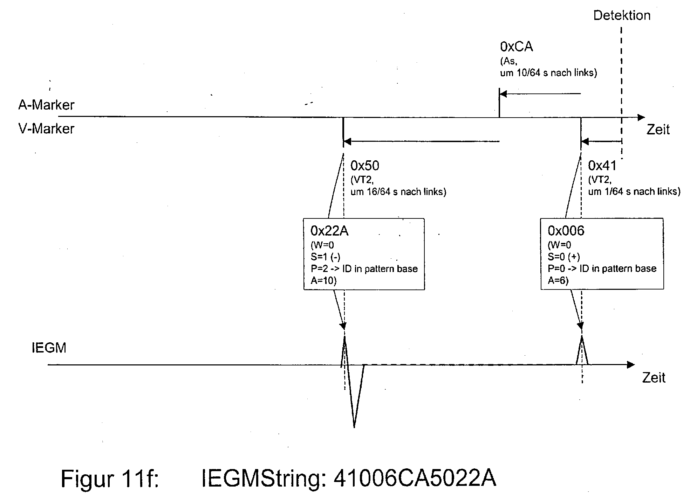

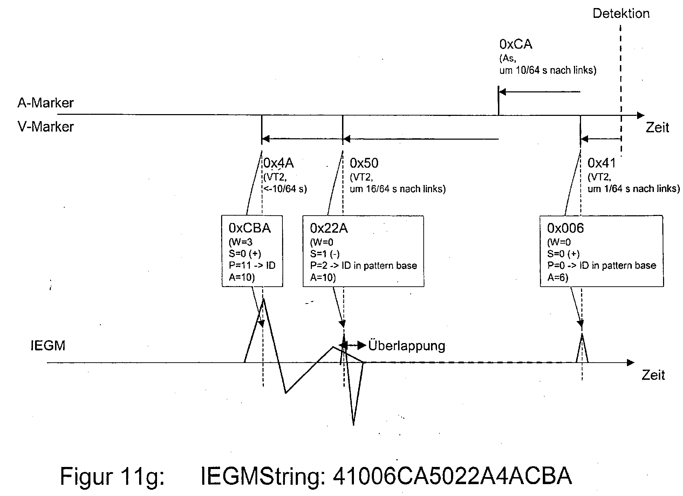

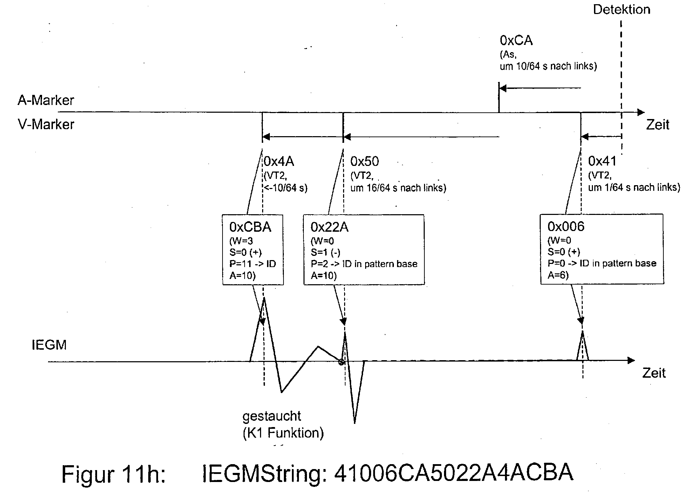

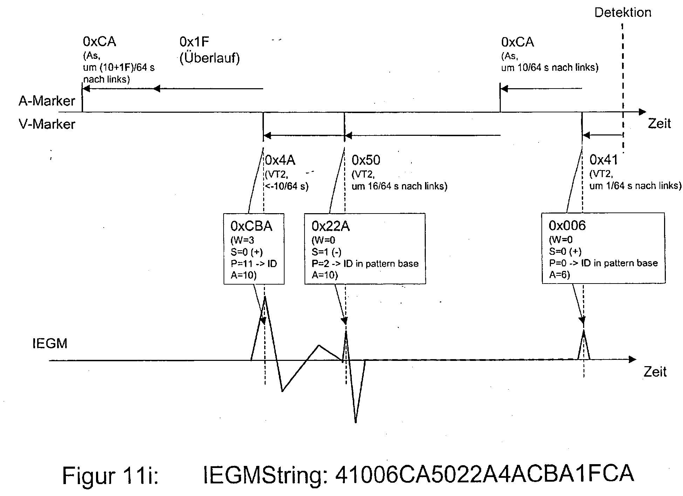

Das Rekonstruktionsverfahren folgt der Beschreibung des Datenstromaufbaus. Ein Beispiel mit verschiedenen Fallunterscheidungen, wie in der konkreten Realisierung ein Datenstrom entschlüsselt wird, ist in Figur 11 dargestellt.

In dem in Figur 11 dargestellten Beispiel wird der Morphologiecode-Datenstrom 41006CA5022A4ACBA1FCA... decodiert und somit das codierte intrakardiale Elektrokardiogramm rekonstruiert.

Die Abfolge von Zeit- (8bit) und Morphologiewerten (12 bit) umfasst insgesamt 47 bytes

*IEGM : = Herzaktion [Herzaktion ...]*

*Herzaktion : = Zeit [V-Morphologie]*

*Zeit := Zeitbyte [Zeitbyte ...]*

Die Abfolge startet immer mit einem Zeitbyte. Die Rekonstruktion startet an der Detektionsmarke (diese wird nicht übertragen) in Richtung Vergangenheit, d.h. der erste Zeitwert spiegelt den Abstand zur Detektionsmarke wider. Ein Zeitbyte hat die folgende 8-Bit Struktur mit Marker bits (M) und Zeit bits (T):

MMMTTTTT

In dem Zeitbyte bedeuten:

TTTTT < 0x1F:       ein Zeitwert in Schritten von 1/64 s, also wie weit aktueller Marker vor dem zuletzt Rekonstruierten zurückliegt

TTTTT ==0x1F:      das Zeitbyte ist als Sonderzeichen zu interpretieren

Die Markerbits (MMM) bedeuten:

000: Vs
001: VT1
010: VT2
011: VF
100: SVT
101: Vp
110: As
111: Ap

Die Sonderzeichen bedeuten:

- Ox1F: Zeitüberlauf, es folgen solange Zeitbytes bis TTTTT<0x1F Gesamtzeitwert = [(Anzahl_Zeitbytes - 1) * 0x1F + TTTTT(<Ox1F)] * (1/64 s)

- 0x3F: Ende der IEGM V-Daten

- 0x5F: Ende der IEGM A-Daten

Der Morphologiecode-Datenstrom ist von links nach rechts zu lesen und ergibt ein Elektrokardiogramm, welches von rechts nach links zu lesen ist.

Zur graphischen (approximativen) Rekonstruktion der Signalverläufe eines Ereignisses wird mittels des im morphologischen Deskriptor übertragenen Mustercodes (P) das entsprechende graphische Muster aus der Musterbasis entnommen. In der Musterbasis sind die Muster sowohl hinsichtlich der Amplitudenachse als auch der Zeitachse normiert dargestellt. Dieses Muster wird dann den Angaben des morphologischen Deskriptors entsprechend skaliert und zwar streckt oder staucht SAAAA die Amplitudenachse (inkl. Rekonstruktion der Polarität) bzw. WW die Zeitachse (Figur 9). Damit wird aus dem neutralen Muster eine Approximation für das originale Ereignis (Signalsegment) erstellt. Ein entscheidender Vorteil gegenüber korrelationsbasierter Musteranalysen (z.B. Correlation Waveform Analysis (CWA)) besteht in der Skalierbarkeit des Musters entlang der Zeitachse. Die Methode der Wavelet-Transformation würde diesen Vorteil zwar auch haben, letztere ist jedoch wesentlich aufwendiger zu realisieren, als das vorgestellte Verfahren.

Die Breitenverhältnisse der einzelnen Zacken werden der Musterbasis entnommen. Für die konkrete Realisierung wurden sie in einer repräsentativen Studie auf heuristischer Basis für jede der einzelnen Muster ermittelt. Liegt eine Codierung in Analogie zu den Amplitudenmustern vor, ist die Decodierung der Breitenverhältnisse in ähnlicher Weise vorzunehmen.

Die Muster werden gemäß ihrer zeitlichen Abfolge aneinandergereiht. Mögliche Überlappungskonflikte infolge der endlich genau klassifizierten Zeit- und Breitenangaben, die nur eine bedingt genaue Rekonstruktion ermöglichen, werden durch Stauchung (wie in Anhang 1 demonstriert) oder Verschmelzung von benachbarten Ereignissen gelöst. Kontext-abhängige Korrekturen (z.B. liegen bei stimulierten Ereignissen typische Latenzzeiten vor) können durch entsprechende Rekonstruktionsregeln (z.B. über ein Expertensystem) bewerkstelligt werden.

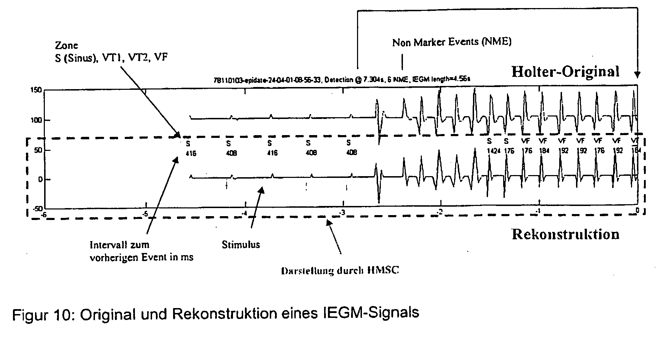

Figur 10 zeigt anhand eines Beispiels den Vergleich zwischen Rekonstruktion und Originalsignal. Das rekonstruierte Signal steht beispielsweise in einem Home-Monitoring-Service-Center (HMSC) zur Verfügung.

Terminologie

| | |
|---|---|
| Kardiales Ereignis: | Signalabschnitt mit überschwelliger Signalenergie, i.a. ein Ausschnitt der Periode eines quasi-periodischen (z.B. im Herzrhythmus pulsierenden) Messsignals (z.B. QRS-Komplex). |
| Ereignissequenz: | chronologisch aufeinanderfolgende Einzelereignisse |
| Marker Event (ME): | Ereignis innerhalb eines definierten Fensters um einen Marker |
| Non Marker Event (NME): | Herzaktivität außerhalb der ME-Fenster |
| Nullbanddurchgang: | Das Nullband erstreckt sich über einem Amplitudenbereich von +/- delta um die Nulllinie. Ein Nullbanddurchgang findet statt, wenn der Signalverlauf von Werten betragsmäßig größer delta einer bestimmten Polarität zu Werten betragsmäßig größer delta der entgegengesetzten Polarität wechselt. Ein Nullbanddurchgang findet hingegen nicht statt, wenn das Signal nur in das Nullband eintaucht, selbst wenn es dabei die Polarität wechselt. |

**Patentansprüche**

1. Signalverarbeitungsvorrichtung
   mit einer Eingangsstufe zur Aufbereitung eines physiologischen Signals, insbesondere eines Elektrokardiogramms, die
   zur Bereitstellung eines aus dem physiologischen Signal gebildeten auszuwertenden Signals mit einer alternierenden Polarität ausgebildet und
   zur Weitergabe des auszuwertenden Signals
   mit einer Auswertungsstufe verbunden ist,
   wobei die Auswertungsstufe ausgebildet ist,
   auf ein dem physiologischen Signal zugeordneten Bezugszeitpunkt anzusprechen und
   für wenigstens ein dem Bezugszeitpunkt zeitlich zugeordnetes Signalsegment des auszuwertenden Signals jeweils
   einen Amplitudenwert für wenigstens einige in dem Signalsegment enthaltene charakteristische Kurvenpunkte, wie Signalmaxima und -minima, Inflexionspunkte oder dergleichen zu erfassen und
   aus den so gewonnenen Amplitudenwerten einen die Form des jeweiligen Signalsegmentes beschreibenden Morphologiecode zu generieren, der eine approximierte Rekonstruktion der Signalmorphologie eines jeweiligen Signalsegmentes erlaubt,
   **dadurch gekennzeichnet, dass**
   die Auswertungsstufe wenigstens einen Nulldurchgangsdetektor aufweist, der ausgebildet ist, Nulldurchgänge des auszuwertenden Signals innerhalb eines Signalsegmentes zu detektieren sowie
   einen Kurvenpunkt-Detektor für charakteristische Kurvenpunkte, wie Signalmaxima und -minima und/oder Inflexionspunkte etc. aufweist, der ausgebildet ist, einen jeweiligen charakteristischen Kurvenpunkt des auszuwertenden Signals zwischen zwei Nulldurchgängen hinsichtlich dessen beschreibender Kennwerte, wie Amplitude und relativer Position auf einer Zeitachse bezüglich eines Referenzsignales zu erfassen
   und wobei
   die Auswertungsstufe ausgebildet ist, aus dem Ausgangssignal des Maximum/Minimumdetektors ein Auswertungssignal zu erzeugen, welches
   auf eine Referenzsignalamplitude bezogene relative Amplituden der Signalmaxima und -minima und
   einen Skalierungsfaktor widerspiegelt und
   welches die die Morphologie beschreibenden Kennwerte widerspiegelt und den Morphologiecode wenigstens teilweise bestimmt.

**2.** Signalverarbeitungsvorrichtung nach Anspruch 1, **dadurch gekennzeichnet, dass** der dem physiologischen Signal zugeordnete Bezugszeitpunkt der Zeitpunkt des Auftretens eines sich in dem physiologischen Signal widerspiegelnden physiologischen Ereignisses ist.

**3.** Signalverarbeitungsvorrichtung nach Anspruch 2, **dadurch gekennzeichnet, dass** die Eingangsstufe zum Verarbeiten von Elektrokardiogrammen ausgebildet ist und die Auswertungsstufe ausgebildet ist, auf den Zeitpunkt eines ventrikulären Ereignisses, insbesondere auf eine R-Zacke in dem Elektrokardiogramm als dem Bezugszeitpunkt anzusprechen.

**4.** Signalverarbeitungsvorrichtung nach einem der Ansprüche 1 bis 3,
**dadurch gekennzeichnet, dass** der Kurvenpunkt-Detektor ein Maximum/Minimumdetektor ist, der ausgebildet ist, ein jeweiliges Signalmaximum oder - minimum des auszuwertenden Signals zwischen zwei Nulldurchgängen hinsichtlich Amplitude und relativer Position auf einer Zeitachse bezüglich eines Referenzsignales zu erfassen.

**5.** Signalverarbeitungsvorrichtung nach einem der Ansprüche 1 bis 4,
**dadurch gekennzeichnet, dass** die Auswertungsstufe ausgebildet ist, aus dem Ausgangssignal des Kurvenpunkt-Detektors bzw. des Maximum/Minimumdetektors ein Auswertungssignal zu erzeugen, welches zusätzlich die relative, auf den Bezugszeitpunkt bezogene zeitliche Position der charakteristischen Kurvenpunkte, insbesondere der Signalmaxima und -minima widerspiegelt.

**6.** Signalverarbeitungsvorrichtung nach einem der Ansprüche 1 bis 5,
**dadurch gekennzeichnet, dass** die Auswertungsstufe ausgebildet ist, das auszuwertende Signal segmentweise derart auszuwerten, dass jeweils ein Signalsegment einen vor dem Bezugszeitpunkt beginnenden und nach dem Bezugszeitpunkt endenden Zeitraum umfasst, wobei der Zeitraum derart festgelegt ist, dass er kürzer ist als die Zeitdauer zwischen zwei aufeinanderfolgenden Bezugszeitpunkten, so dass sich benachbarte Signalsegmente nicht überlappen.

**7.** Signalverarbeitungsvorrichtung nach Anspruch 6,
**dadurch gekennzeichnet, dass** die Eingangsstufe oder die Auswertungsstufe ausgebildet ist, das physiologische Signal derart zu segmentieren, dass ein Signalsegment ein einem wiederkehrenden physiologischen Ereignis zuzuordnendes Signal umfasst.

**8.** Signalverarbeitungsvorrichtung nach Anspruch 1 bis 3 und 6 oder 7,
**dadurch gekennzeichnet, dass** die Auswertungsstufe ausgebildet ist, für jedes Signalsegment einen jeweiligen Morphologiecode in Abhängigkeit des Auswertungssignals für das jeweilige Signalsegment zu generieren.

**9.** Signalverarbeitungsvorrichtung nach einem der Ansprüche 1 bis 8, **dadurch gekennzeichnet, dass**
das Referenzsignal das dem wiederkehrenden physiologischen Ereignis zuzuordnende Signal, insbesondere eine R-Zacke ist,
die Referenzsignalamplitude der numerische Wert der Amplitude des Referenzsignals in dem jeweiligen Signalsegment und
der Referenzsignalzeitpunkt der dem Referenzsignal als physiologischem Signal zugeordnete Zeitpunkt des Auftretens des Referenzsignals in dem Signalsegment.

**10.** Signalverarbeitungsvorrichtung nach Anspruch 9,
**dadurch gekennzeichnet, dass** die Auswertungsstufe ausgebildet ist, auf einen jeweiligen Referenzsignalzeitpunkt als Bezugszeitpunkt anzusprechen.

**11.** Signalverarbeitungsvorrichtung nach einem der Ansprüche 1 bis 10,
**dadurch gekennzeichnet, dass** die Auswertungseinheit mit einem Markersignalgenerator verbunden ist, der ausgebildet ist, ein physiologisches Signal auszuwerten und ein Markersignal zu generieren, welches jeweils ein dem physiologischen Signal zuzuordnendes Ereignis kennzeichnet, wobei die Auswertungseinheit ausgebildet ist, auf den durch ein Markersignal jeweils gegebenen Zeitpunkt als jeweiligen Bezugszeitpunkt anzusprechen.

**12.** Signalverarbeitungsvorrichtung nach einem der Ansprüche 1 bis 11,
**dadurch gekennzeichnet, dass** die Auswertungsstufe ausgebildet ist, zur Bildung des Morphologiecodes vorzugsweise die Kennwerte der charakteristischen Kurvenpunkte bzw. wenigstens die relativen Amplituden der Signalmaxima und -minima ihrer relativen Größe in Bezug auf die Referenzsignalamplitude entsprechend in wenige Amplitudenklassen zu klassifizieren den Morphologiecode derart zu bilden, dass der Morphologiecode einen Mustersubcode enthält, der wenige Werte annehmen kann und die Zugehörigkeit der jeweiligen Amplitude eines Signalmaximums und - minimums zu einer der Amplitudenklassen widerspiegelt.

**13.** Signalverarbeitungsvorrichtung nach Anspruch 12,
**dadurch gekennzeichnet, dass** die Auswertungsstufe ausgebildet ist, die Zugehörigkeit der Amplitude eines Signalmaximums und -minimums zu einer der Amplitudenklassen in Abhängigkeit davon zu bestimmen, ob die jeweilige Amplitude eines Signalmaximums oder -minimums im Verhältnis zur Referenzsignalamplitude ähnlich groß, also genauso groß oder wenig kleiner, mittelgroß oder klein ist.

**14.** Signalverarbeitungsvorrichtung nach Anspruch 12 oder 13,
**dadurch gekennzeichnet, dass** die Auswertungsstufe ausgebildet ist, die relativen Amplituden der Signalmaxima und -minima in Bezug auf den Referenzsignalwert durch einen Ereigniscode wiederzugeben, der für jedes Signalmaximum und - minimum eine Amplituden-Ziffer (□) enthält (i.a. eine Kennwert-Ziffer(□)), die einen von wenigen, der Anzahl der Amplitudenklassen entsprechenden, vorzugsweise vier Werten annehmen kann, und welche die Zugehörigkeit des jeweiligen Signalmaximums oder -minimums zu einer der Amplitudenklassen eindeutig beschreibt.

**15.** Signalverarbeitungsvorrichtung nach einem der Ansprüche 12 bis 14,
**dadurch gekennzeichnet, dass** die Auswertungsstufe ausgebildet ist, ein absolutes Signalmaximum oder -minimum innerhalb eines Signalsegmentes zu bestimmen und den entsprechenden Signalwert als Referenzsignalamplitude zu verwenden.

**16.** Signalverarbeitungsvorrichtung nach Anspruch 15,
**dadurch gekennzeichnet, dass** die Auswertungsstufe ausgebildet ist, einen Ereigniscode zu bilden, der wenige, vorzugsweise vier Amplitudenziffern enthält, die jeweils die Amplitude von vier links und/oder rechts dem Referenzsignal benachbarten Signalmaxima oder -minima innerhalb des jeweiligen Signalsegmentes widerspiegeln.

**17.** Signalverarbeitungsvorrichtung nach Anspruch 16,
**dadurch gekennzeichnet, dass** die Auswertungsstufe ausgebildet ist, aus dem Ereigniscode einen Mustersubcode (PPPPP) zu bilden, indem einer oder mehrere Ereigniscodes über eine Look-Up-Tabelle einem Mustersubcodewert zugeordnet werden.

**18.** Signalverarbeitungsvorrichtung nach Anspruch 16 oder 17,
**dadurch gekennzeichnet, dass** die Auswertungsstufe ausgebildet ist, einen Mustersubcode des Morphologiecodes derart zu bilden, dass der Mustersubcode eine Vorzeichen-Ziffer (S) umfasst, die das Vorzeichen der Referenzsignalamplitude widerspiegelt.

**19.** Signalverarbeitungsvorrichtung nach einem der Ansprüche 1 bis 18,
**dadurch gekennzeichnet, dass** die Auswertungseinheit den Morphologiecode derart bildet, dass der Morphologiecode einen Signalbreiten-Subcode (WW) enthält.

**20.** Signalverarbeitungsvorrichtung nach Anspruch 19,
**dadurch gekennzeichnet, dass** die Auswertungsstufe ausgebildet ist, den Signalbreiten-Subcode (WW) derart zu bilden, dass der Signalbreiten-Subcode (WW) entweder den zeitlichen Abstand zwischen einem ersten Signalmaximum oder -minimum und dem letzten Signalmaximum oder -minimum innerhalb eines Signalsegmentes oder die Dauer eines Signalsegmentes repräsentiert.

**21.** Signalverarbeitungsvorrichtung nach Anspruch 15 und einem der übrigen Ansprüche 1 bis 20,
**dadurch gekennzeichnet, dass** die Auswertungseinheit den Morphologiecode derart bildet, dass der Morphologiecode einen Skalierungs-Subcode (AAAA) enthält, der den Skalierungsfaktor bildet und der die Referenzsignalamplitude als Absolutwert widerspiegelt.

**22.** Signalverarbeitungsvorrichtung nach einem der Ansprüche 1 bis 21,
**dadurch gekennzeichnet, dass** die Auswertungseinheit den Morphologiecode derart bildet, dass der Morphologiecode ein Binärcode ist, der einen oder mehrere der folgenden Bestandteile (Subcodes) aufweist:

einen Signalbreiten-Subcode (WW), der vorzugsweise wenigstens zwei Binärstellen umfasst,

einen Vorzeichen-Subcode (S), der vorzugsweise eine Binärstelle umfasst,
einen Muster-Subcode (PPPPP), der vorzugsweise wenigstens fünf Binärstellen umfasst, und
einen Skalierungs-Subcode (AAAA), der vorzugsweise wenigstens vier Binärstellen umfasst.

23. Signalverarbeitungsvorrichtung nach einem der Ansprüche 1 bis 22,
**dadurch gekennzeichnet, dass** die Auswertungseinheit ausgebildet ist dem Morphologiecode einen Zeitcode zuzuordnen und hinzuzufügen.

24. Signalverarbeitungsvorrichtung nach einem der Ansprüche 1 bis 23,
**dadurch gekennzeichnet, dass** die Eingangsstufe einen Bandpassfilter aufweist, der ausgebildet ist aus dem physiologischen Signal ein differenziertes auszuwertendes Signal zu erzeugen.

25. Implantierbares elektromedizinisches Gerät, insbesondere Herzschrittmacher, mit einer Signalverarbeitungsvorrichtung nach einem der Ansprüche 1 bis 23 sowie mit einer Telemetrieeinheit, die eingangseitig mit der Signalverarbeitungsvorrichtung verbunden und ausgebildet ist, den Morphologiecode drahtlos an ein externes Gerät zu übertragen.

**Claims**

1. A signal processing device
with an input stage to condition a physiological signal, especially an electrocardiogram signal, wherein the input stage is designed to form, from the physiological signal, an alternating polarity signal and provide it for evaluation, and is, in order to transmit the signal to be evaluated
connected with an evaluation stage,
the evaluation stage being designed
to respond to a reference time point associated with the physiological signal, and
to measure one amplitude value for each of at least some characteristic curve points, such as signal maxima and minima, points of inflexion, or similar points, contained in at least one signal segment in the signal to be evaluated, this signal segment being associated in time with the reference time point; and
to generate, from the amplitude values obtained in this way, a morphology code that describes the shape of the respective signal segment and that allows approximate reconstruction of the signal morphology of a respective signal segment,
**characterized in that**
the evaluation stage has at least one zero crossing detector that is designed to detect zero crossings within a signal segment of the signal to be evaluated, and
a curve point detector for characteristic curve points such as signal maxima and minima and/or points of inflexion, etc., that is designed to analyze a respective characteristic curve point of the signal to be evaluated between two zero crossings for parameters describing it such as amplitude and relative position on a time axis with respect to a reference signal,
and wherein
the evaluation stage is designed to produce, from the output signal of the maximum / minimum detector, an evaluation signal that
reflects the relative amplitudes of the signal maxima and minima with respect to a reference signal amplitude and a scaling factor; and
the parameters describing morphology, and that at least partially determines the morphology code.

2. The signal processing device described in claim 1, **characterized in that** the reference time point associated with the physiological signal is the time point when a physiological event reflected in the physiological signal occurs.

3. The signal processing device described in claim 2, **characterized in that** the input stage is designed to process electrocardiograms, and **in that** the evaluation stage is designed to respond to the time point of a ventricular event, in particular an R wave in the electrocardiogram, as the reference time point.

4. The signal processing device described in one of claims 1 through 3,
**characterized in that** the curve point detector is a maximum / minimum detector that is designed to measure the amplitude and relative position on a time axis, with respect to of a reference signal, of a respective signal maximum or minimum of the signal to be evaluated between two zero crossings.

**5.** The signal processing device described in one of claims 1 through 4, **characterized in that** the evaluation stage is designed to produce, from the output signal of the curve point detector or the maximum / minimum detector, an evaluation signal that additionally reflects the relative time position of the characteristic curve points, in particular the signal maxima and minima, with respect to the reference time point.

**6.** The signal processing device described in one of claims 1 through 5, **characterized in that** the evaluation stage is designed to evaluate the signal to be evaluated in segments in such a way that each signal segment comprises a period of time beginning before the reference time point and ending after the reference time point, the period of time being defined in such a way that that it is shorter than the period of time between two successive reference time points, so that adjacent signal segments do not to overlap.

**7.** The signal processing device described in claim 6, **characterized in that** the input stage or the evaluation stage is designed to segment the physiological signal in such a way that a signal segment comprises a signal associated with a repeating physiological event.

**8.** The signal processing device described in claims 1 through 3 and 6 or 7, **characterized in that** the evaluation stage is designed to generate, for every signal segment, a respective morphology code depending on the evaluation signal for the respective signal segment.

**9.** The signal processing device described in one of claims 1 through 8, **characterized in that** the reference signal is the signal associated with the repeating physiological event, in particular an R wave, the reference signal amplitude is the numerical value of the amplitude of the reference signal in the respective signal segment, and the reference signal time point is the time point associated with the reference signal as a physiological signal when the reference signal occurs in the signal segment.

**10.** The signal processing device described in claim 9, **characterized in that** the evaluation stage is designed to respond to a respective reference signal time point as a reference time point.

**11.** The signal processing device described in one of claims 1 through 10, **characterized in that** the evaluation unit is connected with a marker signal generator that is designed to evaluate a physiological signal and generate marker signals, each of which characterizes an event associated with the physiological signal, the evaluation unit being designed to respond to each time point given by a marker signal as the respective reference time point.

**12.** The signal processing device described in one of claims 1 through 11, **characterized in that** the evaluation unit is designed forming the morphology code preferably involves classifying the parameters of the characteristic curve points, or at least the relative amplitudes of the signal maxima and minima, into a few amplitude classes according to their relative size with respect to the reference signal amplitude and forming the morphology code in such a way that it contains a pattern subcode that can assume few values reflecting the amplitude class to which the respective amplitude of a signal maximum and minimum belongs.

**13.** The signal processing device described in claim 12, **characterized in that** the evaluation stage is designed to classify the amplitude of a signal maximum and minimum into one of the amplitude classes as a function of whether the respective amplitude of a signal maximum or minimum is of a size similar to the reference signal amplitude, that is, exactly the same size or a little smaller, medium-sized, or small.

**14.** The signal processing device described in claim 12 or 13, **characterized in that** the evaluation stage is designed to represent the relative amplitudes of the signal maxima and minima with respect to the reference signal value by an event code that contains, for each signal maximum and minimum, an amplitude number (□) (generally speaking a parameter number (□)), that can assume one of a few values, preferably four, corresponding to the number of amplitude classes, and that uniquely describes the amplitude class to which the respective signal maximum or minimum belongs.

**15.** The signal processing device described in one of claims 12 through 14, **characterized in that** the evaluation stage is designed to determine an absolute signal maximum or minimum within a signal segment, and to use the corresponding signal value as a reference signal amplitude.

16. The signal processing device described in claim 15,
**characterized in that** the evaluation stage is designed to form an event code that contains a few, preferably four, amplitude numbers, each of which reflects the amplitude of four adjacent signal maxima or minima to the left and/or to the right of the reference signal within the respective signal segment.

17. The signal processing device described in claim 16,
**characterized in that** the evaluation stage is designed to form, from the event code, a pattern subcode (PPPPP), by assigning one or more event codes to a pattern subcode value through a look-up table.

18. The signal processing device described in claim 16 or 17,
**characterized in that** the evaluation stage is designed to form a pattern subcode of the morphology code in such a way that the pattern subcode comprises a sign number (S) that reflects the sign of the reference signal amplitude.

19. The signal processing device described in one of claims 1 through 18,
**characterized in that** the evaluation unit forms the morphology code so that it contains a signal width subcode (WW).

20. The signal processing device described in claim 19,
**characterized in that** the evaluation stage is designed to form the signal width subcode (WW) in such a way that the signal width subcode (WW) represents either the time interval between a first signal maximum or minimum and the last signal maximum or minimum within a signal segment, or the duration of a signal segment.

21. The signal processing device described in claim 15 and in one of the other claims 1 through 20,
**characterized in that** the evaluation unit forms the morphology code in such a way that the morphology code contains a scaling subcode (AAAA) that the forms the scaling factor and that reflects the reference signal as an absolute value.

22. The signal processing device described in one of claims 1 through 21,
**characterized in that** the evaluation unit forms the morphology code in such a way that the morphology code is a binary code that has one or more of the following components (subcodes):

a signal width subcode (WW) that preferably comprises at least two bits,
a sign subcode (S) that preferably comprises one bit,
a pattern subcode (PPPPP) that preferably comprises at least five bits, and
a scaling subcode (AAAA) that preferably comprises at least four bits.

23. The signal processing device described in one of claims 1 through 22, **characterized in that** the evaluation unit is designed to assign and add a time code to the morphology code.

24. The signal processing device described in one of claims 1 through 23,
**characterized in that** the input stage has a bandpass filter that is designed to produce, from the physiological signal a differentiated signal to be evaluated.

25. An implantable electromedical device, especially a cardiac pacemaker, with the signal processing device described in one of claims 1 through 23 and with a telemetry unit that is connected, on the input side, with the signal processing device and that is designed to transfer the morphology code to an external device using wireless means.

**Revendications**

1. Dispositif de traitement de signal
avec un niveau d'entrée pour la préparation d'un signal physiologique, notamment, un électrocardiogramme, qui est conçu pour la fourniture d'un signal avec une polarité alternée à exploiter formé à partir du signal physiologique et est relié avec un niveau d'exploitation
pour la retransmission du signal à exploiter,
où le niveau d'exploitation est conçu pour
réagir à un instant de référence associé au signal physiologique et

saisir, pour au moins un segment de signal du signal à exploiter associé dans le temps à l'instant de référence, chaque fois une valeur d'amplitude pour au moins quelques points de courbe caractéristiques contenus dans le segment de signal, tels que des maxima et des minima de signal, des points d'inflexion ou similaires, et

générer un code de morphologie décrivant la forme du segment de signal respectif à partir des valeurs d'amplitude ainsi obtenues, qui permet une reconstruction approximative de la morphologie du signal d'un segment de signal respectif,

**caractérisé en ce que**

le niveau d'exploitation présente au moins un détecteur de passage par zéro, qui est conçu pour détecter les passages par zéro du signal à exploiter à l'intérieur du segment de signal, ainsi qu'un

détecteur de points de la courbe pour des points de la courbe caractéristiques, tels que des maxima de signal et des minima de signal et/ou des points d'inflexion etc., qui est conçu pour saisir un point de la courbe caractéristique respectif du signal à exploiter entre deux passages par zéro de ses valeurs de références le décrivant, telles que l'amplitude et la position relative sur un axe de temps par rapport à un signal de référence,

et où

le niveau d'exploitation est conçu pour générer un signal d'évaluation à partir du signal de sortie du détecteur de maximum/ de minimum, lequel

reflète des amplitudes relatives des maxima et des minima de signal par rapport à une amplitude du signal de référence et

un facteur d'échelle, et

lequel reflète les valeurs de référence décrivant la morphologie et détermine au moins partiellement le code de morphologie.

2. Dispositif de traitement d'un signal selon la revendication 1, **caractérisé en ce que** l'instant de référence associé au signal physiologique est l'instant de l'apparition d'un évènement physiologique se reflétant dans le signal physiologique.

3. Dispositif de traitement de signal selon la revendication 2, **caractérisé en ce que** le niveau d'entrée est conçu pour le traitement des électrocardiogrammes et le niveau d'exploitation est conçu pour réagir au moment d'un évènement ventriculaire, notamment suite à une onde R dans l'électrocardiogramme servant d'instant de référence.

4. Dispositif de traitement de signal selon l'une des revendications 1 à 3,
**caractérisé en ce que** le détecteur de points de la courbe est un détecteur de maximum/ de minimum qui est conçu pour saisir un maximum ou un minimum de signal respectif du signal à exploiter entre deux passages par zéro au niveau de l'amplitude et de la position relative sur un axe de temps par rapport à un signal de référence.

5. Dispositif de traitement de signal selon l'une des revendications 1 à 4,
**caractérisé en ce que** le niveau d'exploitation est conçu pour générer un signal d'exploitation à partir du signal de sortie du détecteur de points de la courbe, respectivement, du détecteur de maximum/de minimum, lequel reflète de manière complémentaire la position relative dans le temps des points de la courbe caractéristiques, notamment le maxima et le minima de signal, par rapport à l'instant de référence.

6. Dispositif de traitement de signal selon l'une des revendications 1 à 5,
**caractérisé en ce que** le niveau d'exploitation est conçu pour exploiter de manière segmentée le signal à exploiter de telle manière que chaque segment de signal comprend un moment commençant avant l'instant de référence et terminant après l'instant de référence, où le moment est fixé de telle façon qu'il est plus court que la durée entre deux points de référence se succédant de sorte que des segments de signaux voisins ne se superposent pas.

7. Dispositif de traitement de signal selon la revendication 6,
**caractérisé en ce que** le niveau d'entrée ou le niveau d'exploitation sont conçus pour segmenter le signal physiologique de telle façon qu'un segment de signal comprend un signal associé à un évènement physiologique récurrent.

8. Dispositif de traitement de signal selon les revendications 1 à 3 et 6 ou 7,
**caractérisé en ce que** le niveau d'exploitation est conçu pour générer, pour chaque segment de signal, un code de morphologie respectif en fonction du signal d'exploitation pour le segment de signal en question.

9. Dispositif de traitement de signal selon l'une des revendications 1 à 8, **caractérisé en ce que**
le signal de référence est le signal associé à l'évènement physiologique récurrent, est notamment une onde R, l'amplitude du signal de référence est la valeur numérique de l'amplitude du signal de référence dans le segment de signal respectif, et

l'instant du signal de référence est l'instant de l'apparition du signal de référence dans le segment de signal associé au signal de référence en tant que signal physiologique.

10. Dispositif de traitement de signal selon la revendication 9,
**caractérisé en ce que** le niveau d'exploitation est conçu pour réagir à l'instant du signal de référence respectif en tant que point de référence.

11. Dispositif de traitement de signal selon l'une des revendications 1 à 10,
**caractérisé en ce que** l'unité d'exploitation est reliée avec un générateur de signaux de marquage qui est conçu pour exploiter un signal physiologique et générer un signal de marquage lequel caractérise respectivement un évènement associé au signal physiologique, où l'unité d'exploitation est conçue pour réagir à l'instant respectivement indiqué par un signal de marquage en tant qu'instant de référence.

12. Dispositif de traitement de signal selon l'une des revendications 1 à 11,
**caractérisé en ce que** le niveau d'exploitation est prévu, pour la formation du code de morphologie, pour classer de manière correspondante en classes d'amplitudes, de préférence les valeurs de références des points de la courbe caractéristiques, respectivement, au moins les amplitudes relatives des maxima et des minima de signal, en fonction de leur taille relative par rapport à l'amplitude du signal de référence et former le code de morphologie de telle façon que le code de morphologie contienne un sous-code modèle qui peut admettre peu de valeurs et reflète l'appartenance de l'amplitude respective d'un maximum et d'un minimum de signal à une des classes d'amplitude.

13. Dispositif de traitement de signal selon la revendication 12,
**caractérisé en ce que** le niveau d'exploitation est conçu pour déterminer l'appartenance de l'amplitude d'un maximum et d'un minimum de signal à l'une des classes d'amplitudes en fonction du fait que l'amplitude respective d'un maximum ou d'un minimum de signal est de taille semblable, exactement de même taille ou un peu plus petite, de taille moyenne ou petite par rapport à l'amplitude du signal de référence.

14. Dispositif de traitement de signal selon les revendications 12 ou 13,
**caractérisé en ce que** le niveau d'exploitation est conçu pour reproduire les amplitudes relatives des maxima et minima de signal en tenant compte de la valeur du signal de référence par un code évènementiel qui contient un chiffre d'amplitude ($\square$) (en général, un chiffre identifiant ($\square$)) pour chaque maximum et minimum de signal, qui peut adopter une valeur parmi peu de valeurs, de préférence quatre, correspondant au nombre des classes d'amplitudes et lequel décrit de manière évidente l'appartenance du maximum ou du minimum de signal respectif à l'une des classes d'amplitudes.

15. Dispositif de traitement de signal selon l'une des revendications 12 à 14,
**caractérisé en ce que** le niveau d'exploitation est conçu pour déterminer un maximum ou un minimum de signal absolu dans un segment de signal et utiliser la valeur de signal correspondante en tant qu'amplitude de signal de référence.

16. Dispositif de traitement de signal selon la revendication 15,
**caractérisé en ce que** le niveau d'exploitation est conçu pour former un code évènementiel qui contient peu, de préférence, quatre, chiffres d'amplitudes qui reflètent respectivement les amplitudes de quatre maxima ou minima de signal voisins à gauche et/ou à droite du signal de référence dans le segment de signal respectif.

17. Dispositif de traitement de signal selon la revendication 16,
**caractérisé en ce que** le niveau d'exploitation est conçu pour former un sous-code modèle (PPPPP) à partir du code évènementiel, **en ce qu'**un ou plusieurs codes évènementiels sont associés à une valeur de sous-code modèle par un tableau de consultation.

18. Dispositif de traitement de signal selon les revendications 16 ou 17,
**caractérisé en ce que** le niveau d'exploitation est conçu pour former un sous-code modèle du code de morphologie de telle façon que le sous-code modèle comprenne un chiffre de signe précurseur (S) qui reflète le signe précurseur de l'amplitude du signal de référence.

19. Dispositif de traitement de signal selon l'une des revendications 1 à 18,

**caractérisé en ce que** l'unité d'exploitation forme le code de morphologie de telle façon que le code de morphologie contienne un sous-code de largeur de signal (WW).

20. Dispositif de traitement de signal selon la revendication 19,
**caractérisé en ce que** le niveau d'exploitation est conçu pour former le sous-code de largeur de signal (WW) de telle façon que le sous-code de largeur de signal (WW) représente soit le temps s'écoulant entre un premier maximum, ou minimum, de signal et le dernier maximum, ou minium, de signal dans un segment de signal, ou la durée d'un segment de signal.

21. Dispositif de traitement de signal selon la revendication 15, et l'une des autres revendications de 1 à 20,
**caractérisé en ce que** l'unité d'exploitation forme le code de morphologie de telle façon que le code de morphologie contienne un sous-code d'échelle (AAAA) qui forme le facteur d'échelle et reflète l'amplitude du signal de référence en tant que valeur absolue.

22. Dispositif de traitement de signal selon l'une des revendications 1 à 21,
**caractérisé en ce que** l'unité d'exploitation forme le code de morphologie de telle façon que le code de morphologie est un code binaire qui présente un ou plusieurs des composants suivants (sous-codes) :

un sous-code de largeur de signal (WW) qui comprend de préférence au moins deux chiffres binaires,
un sous-code de signe précurseur (S) qui comprend de préférence un chiffre binaire,
un sous-code modèle (PPPPP) qui comprend de préférence au moins cinq chiffres binaires, et
un sous-code d'échelle (AAAA) qui comprend de préférence au moins quatre chiffres binaires.

23. Dispositif de traitement de signal selon l'une des revendications 1 à 22,
**caractérisé en ce que** l'unité d'exploitation est conçue pour attribuer et rajouter un code de temps au code de morphologie.

24. Dispositif de traitement de signal selon l'une des revendications 1 à 23,
**caractérisé en ce que** le niveau d'entrée présente un filtre de bande passante qui est conçu pour générer un signal différentié à exploiter à partir du signal physiologique.

25. Appareil électro-médical implantable, notamment stimulateur cardiaque, avec un dispositif de traitement de signal selon l'une des revendications 1 à 23, ainsi qu'avec une unité de télémétrie qui est reliée du côté de l'entrée avec le dispositif de traitement de signal et est conçu pour transmettre sans fil le code de morphologie vers un appareil externe.

• signifikante Signalzacken

**Fig.1** Festlegung signifikanter Signalzacken

**Fig.2** Verteilung von IEGM-Morphologien

**Fig.3**

**Fig.4**

Fig.5

Fig.6

```
              ┌─────────┐    Morphologischen
              (  Start  )    Deskriptor bilden
              └────┬────┘
                   │
        ┌──────────┴──────────┐
        │    Segmentierung    │
        │    des IEGMs in     │
        │   ME, NME Ereignisse│
        └──────────┬──────────┘
                   │
        ┌──────────┴──────────┐
        │  Nullbanddurchgänge │
        │   im Ergebnis finden│
        └──────────┬──────────┘
                   │
┌──────────┐ speichern ┌──────────────────────┐
│ Amplitude│◄──────────│ Signifikante Signalzacken│
│   AAAA   │           │ und globales Extremum finden│
└──────────┘           └──────────┬──────────┘
                                  │
  ME,NME<5 Zacken            Ergebnistyp?      ME,NME >5 Zacken
```

Abstand zwischen
1. und letzter Zacke

Breite ww — speichern

Signalzacken
klassifizieren (k,m,g)

zentrale g-Zacke
finden und deren Signum

Signum S — speichern

Satelliten um zentrale Marke
nach Wichtigkeit sortieren

Ereigniscode $c_e$ berechnen

Mustercode über look-up
Tabelle ermitteln

Muster PPPPP — speichern

Anzahl der Zacken
klassifizieren

MMM
W W
— speichern

Sondercode für
NME eintragen

Muster PPPPP — speichern

Morphologischer Deskriptor

WWSPPPPPAAAA

in Zeitinfo
übernehmen

MMM

**Fig.7**

Marken

IEGM    ME    ME    NME    NME    ME

← 200ms →

Analyse
Fenster

|80ms 20ms|

|100ms|

## Fig.8

IEGM-String

Muster Basis

ID

WWSPPPPPAAAA

Skalierung

1    (xp,yp)

1

-1

Skalierung

$X = K1(w(WW) * X\_P) + K0$    K0,K1: Kontextabhängige Korrekturfunktionen

$Y = Y\_P * (-1)^S * (AAAA)$    Default: K0 = 0 ms, V-pace: K0 = 40 ms

$X\_P = [xp(1), ........, xp(n)]$    w(WW): Abbildung auf die Zentren der Breiteklassen

$Y\_P = [yp(1), ........, yp(n)]$

## Fig.9

Figur 10: Original und Rekonstruktion eines IEGM-Signals

Detektion

A-Marker

V-Marker                                                             Zeit

IEGM

Zeit

Figur 11a:    IEGMString:

Detektion

A-Marker

V-Marker                                                        Zeit

0x41
(VT2,
um 1/64 s nach links)

IEGM

Zeit

Figur 11b:    IEGMString: 41

Detektion

A-Marker

V-Marker

Zeit

0x41
(VT2,
um 1/64 s nach links)

0x006
(W=0
S=0 (+)
P=0 -> ID in pattern base
A=6)

IEGM

Zeit

## Figur 11c:    IEGMString: 41006

Detektion

0xCA
(As,
um 10/64 s nach links)

A-Marker

V-Marker                                                                 Zeit

0x41
(VT2,
um 1/64 s nach links)

0x006
(W=0
S=0 (+)
P=0 -> ID in pattern base
A=6)

IEGM

Zeit

Figur 11d:     IEGMString: 41006CA

Detektion

0xCA
(As,
um 10/64 s nach links)

A-Marker

V-Marker                                                                          Zeit

0x50
(VT2,
um 16/64 s nach links)

0x41
(VT2,
um 1/64 s nach links)

0x006
(W=0
S=0 (+)
P=0 -> ID in pattern base
A=6)

IEGM

Zeit

Figur 11e:     IEGMString: 41006CA50

Detektion

0xCA
(As,
um 10/64 s nach links)

A-Marker

V-Marker                                                                                    Zeit

0x50                                         0x41
(VT2,                                        (VT2,
um 16/64 s nach links)                       um 1/64 s nach links)

| 0x22A |
|---|
| (W=0 |
| S=1 (-) |
| P=2 -> ID in pattern base |
| A=10) |

| 0x006 |
|---|
| (W=0 |
| S=0 (+) |
| P=0 -> ID in pattern base |
| A=6) |

IEGM

                                                                                           Zeit

Figur 11f:      IEGMString: 41006CA5022A

Detektion

0xCA
(As,
um 10/64 s nach links)

A-Marker

V-Marker

Zeit

0x4A
(VT2,
<-10/64 s)

0x50
(VT2,
um 16/64 s nach links)

0x41
(VT2,
um 1/64 s nach links)

| 0xCBA | 0x22A | 0x006 |
|---|---|---|
| (W=3 | (W=0 | (W=0 |
| S=0 (+) | S=1 (-) | S=0 (+) |
| P=11 -> ID | P=2 -> ID in pattern base | P=0 -> ID in pattern base |
| A=10) | A=10) | A=6) |

IEGM

Überlappung

Zeit

Figur 11g:    IEGMString: 41006CA5022A4ACBA

Detektion

0xCA
(As,
um 10/64 s nach links)

A-Marker

V-Marker

Zeit

0x4A
(VT2,
<-10/64 s)

0x50
(VT2,
um 16/64 s nach links)

0x41
(VT2,
um 1/64 s nach links)

0xCBA
(W=3
S=0 (+)
P=11 -> ID
A=10)

0x22A
(W=0
S=1 (-)
P=2 -> ID in pattern base
A=10)

0x006
(W=0
S=0 (+)
P=0 -> ID in pattern base
A=6)

IEGM

Zeit

gestaucht
(K1 Funktion)

Figur 11h:    IEGMString: 41006CA5022A4ACBA

Detektion

0xCA
(As,
um (10+1F)/64 s
nach links)

0x1F
(Überlauf)

0xCA
(As,
um 10/64 s nach links)

A-Marker

V-Marker

Zeit

0x4A
(VT2,
<-10/64 s)

0x50
(VT2,
um 16/64 s nach links)

0x41
(VT2,
um 1/64 s nach links)

0xCBA
(W=3
S=0 (+)
P=11 -> ID
A=10)

0x22A
(W=0
S=1 (-)
P=2 -> ID in pattern base
A=10)

0x006
(W=0
S=0 (+)
P=0 -> ID in pattern base
A=6)

IEGM

Zeit

Figur 11i:    IEGMString: 41006CA5022A4ACBA1FCA

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2836890
- EP 1118307 A
- EP 0601775 U